# EUROPEAN PATENT SPECIFICATION

(11) **EP 4 343 790 B1**
(45) Date of publication and mention of the grant of the patent: **01.07.2026**
(21) Application number: 22915245.9
(22) Date of filing: 30.12.2022
(51) Int. Cl.: G16H 70/20

(54) **MEDICAL EQUIPMENT OPERATION GUIDANCE METHOD AND SYSTEM**
VERFAHREN UND SYSTEM ZUR BETRIEBSFÜHRUNG MEDIZINISCHER AUSRÜSTUNGEN
PROCÉDÉ ET SYSTÈME DE GUIDAGE D'OPÉRATION D'ÉQUIPEMENT MÉDICAL

(30) Priority: 30.12.2021 CN 202111661102
(43) Date of publication of application: 27.03.2024
(73) Proprietor: Shanghai United Imaging Healthcare Co., Ltd., Shanghai 201807 (CN)
(72) Inventor: WANG, Tian, Shanghai 201807 (CN)
(74) Representative: Wang, Bo
(86) International application number: PCT/CN2022/144300
(87) International publication number: WO 2023/125998

(56) References cited:
- CN-A- 106 021 879
- CN-A- 110 033 767
- CN-A- 111 143 004
- CN-A- 113 168 906
- CN-A- 114 255 882
- JP-A- 2012 014 407
- US-A1- 2017 329 903

## Description

### TECHNICAL FIELD

The present disclosure relates to a medical information technology field, in particular, relates to systems and methods for guiding an operation of a medical device.

### BACKGROUND

Magnetic resonance imaging (MRI) is an imaging technique that is commonly used, safe, and radiation free in clinical practice. An MRI device mainly utilizes a principle of magnetic resonance to scan a target object and obtain a magnetic resonance image. However, to complete magnetic resonance scanning and obtain the magnetic resonance image, a complex magnetic resonance system is required, and operations of the magnetic resonance system are also relatively complex, making it difficult for operators to fully grasp details of some functions. When using some uncommon functions of the magnetic resonance system, it is often necessary to consult manuals or manufacturers, resulting in very low device operation efficiency and a poor result. US 2017/329903 A1 relates to methods, devices, and systems for providing operation instruction information of medical apparatuses.

Therefore, it is desirable to provide a method and system for guiding an operation of a medical device to assist the operators in device operation, and improve device operation efficiency and medical examination results.

### SUMMARY

The present invention defined by the appended claims.

### BRIEF DESCRIPTION OF THE DRAWINGS

This specification will be further illustrated by way of exemplary embodiments, which will be described in detail with the accompanying drawings. These examples are non-limiting, and in these examples, the same number indicates the same structure, wherein:
FIG. 1 is a schematic diagram illustrating an exemplary application scenario of a system for guiding an operation of a medical device according to some embodiments of the present disclosure;
FIG. 2 is a block diagram illustrating an exemplary system for guiding an operation of a medical device according to some embodiments of the present disclosure;
FIG. 3 is a flowchart illustrating an exemplary process for guiding an operation of a medical device according to some embodiments of the present disclosure;
FIG. 4A is a schematic diagram illustrating an exemplary information retrieving interface according to some embodiments of the present disclosure;
FIG. 4B is a schematic diagram illustrating an exemplary first information feedback interface according to some embodiments of the present disclosure;
FIG. 4C is a schematic diagram illustrating another exemplary first information feedback interface according to some embodiments of the present disclosure;
FIG. 4D is a schematic diagram illustrating an exemplary second information feedback interface according to some embodiments of the present disclosure;
FIG. 4E is a schematic diagram illustrating another exemplary information retrieving interface according to some embodiments of the present disclosure;
FIG. 4F is a schematic diagram illustrating another exemplary first information feedback interface according to some embodiments of the present disclosure;
FIG. 4G is a schematic diagram illustrating another exemplary second information feedback interface according to some embodiments of the present disclosure;
FIG. 4H is a schematic diagram illustrating another exemplary information retrieving interface according to some embodiments of the present disclosure;
FIG. 4I is a schematic diagram illustrating another exemplary first information feedback interface according to some embodiments of the present disclosure;
FIG. 4J is a schematic diagram illustrating another exemplary second information feedback interface according to some embodiments of the present disclosure;
FIG.5 is a schematic diagram illustrating an exemplary user dialogue window according to some embodiments of the present disclosure;
FIG. 6 is a flowchart illustrating an exemplary process for determining at least one piece of guide information according to some embodiments of the present disclosure;
FIG.7 is a schematic diagram illustrating an exemplary retrieval analysis model according to some embodiments of the present disclosure;
FIG. 8 is a flowchart illustrating another exemplary process for determining at least one piece of guide information according to some embodiments of the present disclosure;
FIG. 9 is a flowchart illustrating another exemplary process for guiding a user in operating on a target operating interface to complete a target task according to some embodiments of the present disclosure;
FIG.10A is a schematic diagram illustrating an exemplary target operating interface according to some embodiments of the present disclosure;
FIG.10B is a schematic diagram illustrating another exemplary target operating interface according to some embodiments of the present disclosure;
FIG.11A is a schematic diagram illustrating an exemplary target operating interface including operating guide information according to some embodiments of the present disclosure;
FIG.11B is a schematic diagram illustrating another exemplary target operating interface including operating guide information according to some embodiments of the present disclosure; and
FIG.11C is a schematic diagram illustrating another exemplary target operating interface including operating guide information according to some embodiments of the present disclosure.

In order to more clearly illustrate the technical solutions of the embodiments of the present specification, the following briefly introduces the drawings that need to be used in the description of the embodiments. Apparently, the accompanying drawings in the following description are only some examples or embodiments of this specification, and those skilled in the art can also apply this specification to other similar scenarios. Unless obviously obtained from the context or the context illustrates otherwise, the same numeral in the drawings refers to the same structure or operation.

It should be understood that "system," "device," "unit" and/or "module" as used herein is a method for distinguishing different components, elements, parts, parts or assemblies of different levels. However, the words may be replaced by other expressions if other words can achieve the same purpose.

As indicated in the specification and claims, the terms "a," "an," "an" and/or "the" are not specific to the singular and may include the plural unless the context clearly indicates an exception. Generally speaking, the terms "comprising" and "comprising" only suggest the inclusion of clearly identified operations and elements, and these operations and elements do not constitute an exclusive list, and the method or device may also contain other operations or elements.

Flowcharts are used in the present disclosure to illustrate the operation performed by the system according to the embodiment of the present disclosure. It should be understood that the preceding or subsequent operations are not necessarily performed accurately in sequence. Instead, the operations may be processed in reverse order or simultaneously. At the same time, other operations may add to these procedures, or remove one or more operations from these procedures.

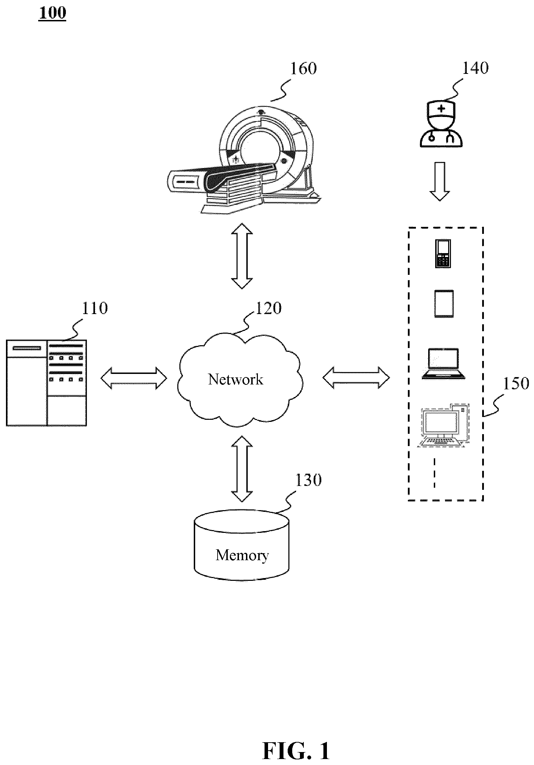

FIG. 1 is a schematic diagram illustrating an exemplary application scenario of a system for guiding an operation of a medical device according to some embodiments of the present disclosure.

As shown in FIG. 1, a system 100 for guiding an operation of a medical device (herein refers to the system 100) includes a processor 110, a network 120, a memory 130, a user 140, an apparatus 150 for guiding an operation of a medical device (herein refers to the apparatus 150), and a medical device 160.

The processor 110 may process data and/or information obtained from various components or other information sources of the system 100. For example, the processor 110 may obtain retrieving information input by the user 140 from the apparatus 150. As another example, the processor 110 may send guide information determined based on the retrieving information to the apparatus 150 for guiding the user 140 in operation. In some embodiments, the processor 110 may be local or remote. For example, the processor 110 may access information and/or data from the memory 130, the apparatus 150, and the medical device 160 through the network 120. As another example, the processor 110 may be directly connected to the memory 130, the apparatus 150, and medical device 160 to access the information and/or data. In some embodiments, the processor 110 and the apparatus 150 may be integrated. In some embodiments, the processor 110 and the apparatus 150 may be directly or indirectly connected to work together to achieve the processes and/or functions described in the present disclosure.

The network 120 includes any suitable network capable of facilitating information and/or data exchange. In some embodiments, at least one component of the system 100 (e.g. the processor 110, the memory 130, the apparatus 150, and the medical device 160) may exchange the information and/or data with at least one other component of the system 100 through the network 120. For example, the processor 110 may obtain a historical operation record of the user from the memory 130 through the network 120.

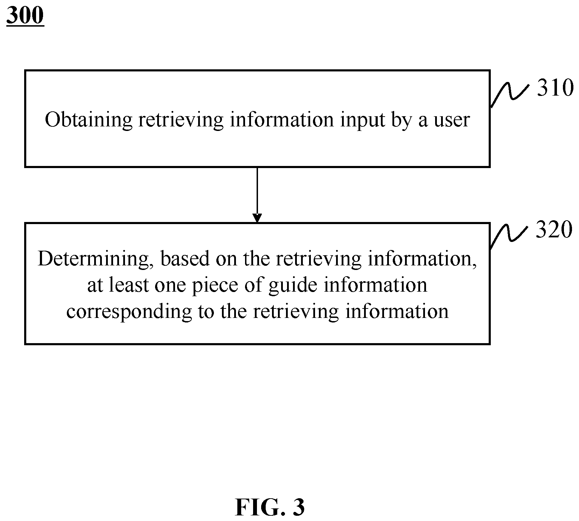

The memory 130 may store data, instructions, and/or any other information related to the system. In some embodiments, the memory 130 may store data and/or information obtained by the processor 110, the apparatus 150, and the medical device 160 (e.g., the retrieving information, the guide information, the historical operation record of the user, etc.). In some embodiments, the memory 130 may store data and/or instructions used by the processor 110 to execute or use to complete the exemplary processes described in the present disclosure. For example, the memory 130 may be equipped with a medical device knowledge base. The medical device knowledge base (e.g., a magnetic resonance imaging knowledge base) may be constructed and updated by device manufacturers of the medical device. The medical device knowledge base includes multiple reference guide information for the medical device, and contents of the reference guide information include but are not limited to principle knowledges of the medical device, operating processes of the medical device (e.g., clinical scanning guidelines), or the like. The forms of reference guide information include but are not limited to a text, a video, an image, or the like. The processor 110 may determining the at least one piece of guide information corresponding to the retrieving information from the medical device knowledge base based on the retrieving information. More description of the guide information may be found in FIG. 3 and related descriptions. In some embodiments, the memory 130 may be part of the processor 110, the apparatus 150, and/or the medical device 160.

The user 140 refers to a user for operating the medical device 160 such as a device maintenance personnel of the medical device 160, operators, or the like.

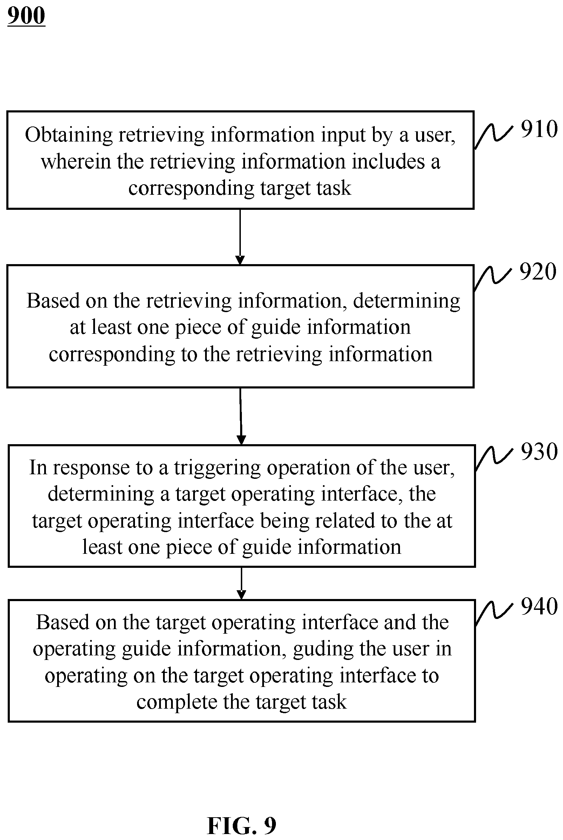

The apparatus 150 refers to an apparatus used to retrieve, display, and view information related to medical device 160. The apparatus 150 may display guide information of the medical device, medical image data generated by scanning of the medical device 160, or the like. In some embodiments, the apparatus 150 may be set as an operating terminal for the medical device (e.g., a magnetic resonance system). In some embodiments, the apparatus 150 may also be a mobile phone, a tablet, a laptop, or any combination thereof. In some embodiments, the apparatus 150 includes a display interface, an operation interface, or the like. In some embodiments, the processor 110 may interact with the user 140 through the apparatus 150. The user 140 may input retrieving information through the apparatus 150 and perform corresponding operations based on the guide information displayed on the apparatus 150. The user 140 may operate a target operating interface based on the guide information displayed on the apparatus 150 to operate the medical device 160 for completing the target task. More descriptions of the target operating interface may be found in FIG. 9 and related descriptions. In some embodiments, the apparatus 150 may communicate with other components in the system 100. For example, the apparatus 150 may communicate with the processor 110 through the network 120 to obtain guide information generated by the processor 110 for processing. As another example, the apparatus 150 may directly communicate with the medical device 160, send operation instructions generated by a user operation to the medical device 160 to execute the target task.

The medical device 160 refers to a related device used to perform medical tasks. The medical device 160 may include but is not limited to a digital imaging device, a magnetic resonance imaging device, a computed tomography (CT) device, an ultrasound imaging device, a nuclear medicine imaging device, or the like. In some embodiments, the medical device 160 may communicate with other components in the system 100. For example, the medical device 160 may communicate with the memory 130 to store medical image data generated by scanning in the memory 130. As another example, the medical device 160 may communicate with the apparatus 150 to obtain corresponding instructions issued by the user 140 through the apparatus 150 to execute a corresponding target task. _{∘}

For example, the application scenario 100 of the system for guiding the operation of the medical device further includes a cloud storage. As another example, the application scenario 100 of the system for guiding the operation of the medical device may be implemented on other devices to achieve similar or different functions. However, these changes and modifications will not deviate from the scope of the present disclosure.

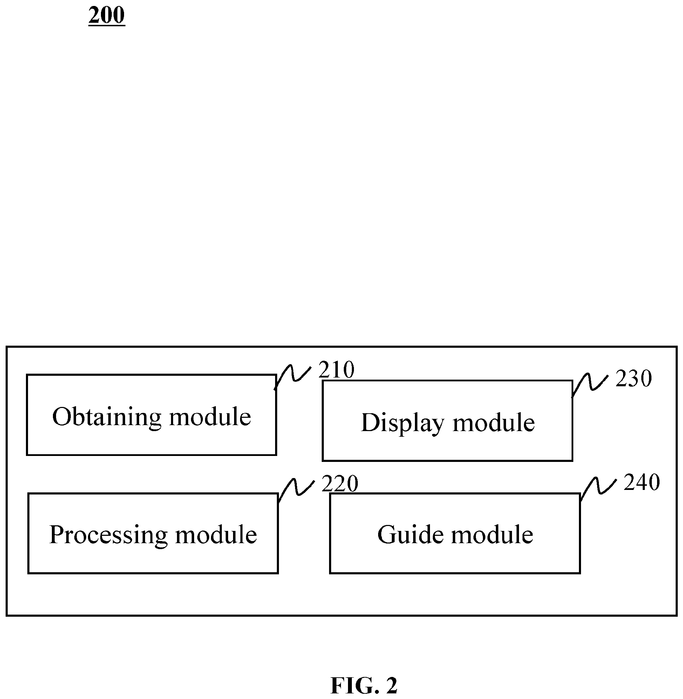

FIG. 2 is a block diagram illustrating an exemplary system for guiding an operation of a medical device according to some embodiments of the present disclosure.

As shown in FIG. 2, a system 200 for guiding an operation of a medical device (herein refers to the system 200) includes an obtaining module 210 and a processing module 220.

The obtaining module 210 is configured to obtain retrieving information input by the user. In some embodiments, when the at least one piece of guide information includes multiple pieces of guide information, the obtaining module 210 is further configured to obtain at least one of a historical operation record of the user, user information, and a current scanning body part.

The processing module 220 is configured to determine, based on the retrieving information, at least one piece of guide information corresponding to the retrieving information. in some embodiments, the processing module 220 is further configured to determine a sequence number for each of the multiple pieces of guide information based on the multiple pieces of guide information and the at least one of the historical operation record, the user information, and the current scanning body part. In some embodiments, when the retrieving information includes the text information and/or the voice information, the processing module 220 is further configured to determine a retrieving keyword of the retrieving information by processing the retrieving information; and determine, based on the retrieving keyword, the at least one piece of guide information corresponding to the retrieving information. In some embodiments, the processing module 220 is further configured to determine the at least one piece of guide information corresponding to the retrieving information by processing, based on a retrieval analysis model, the retrieving information. In some embodiments, the processing module 220 is further configured to determine a task type of the target task based on the retrieving information; in response to determining that the task type of the target task is the fault detection task, obtain a fault interface and one or more system logs of the medical device used by the user; and determine, based on the retrieving information, the fault interface, and the system logs, the at least one piece of guide information corresponding to the retrieving information.

As shown in FIG. 2, the system 200 further includes a display module 230, and in response to a triggering operation of the user, the display module 230 is configured to display a target operating interface.

As shown in FIG. 2, the system further includes a guide module 240, the guide module 240 is configured to guide, based on the operating guide information, the user in operating on the target operating interface to complete the target task.

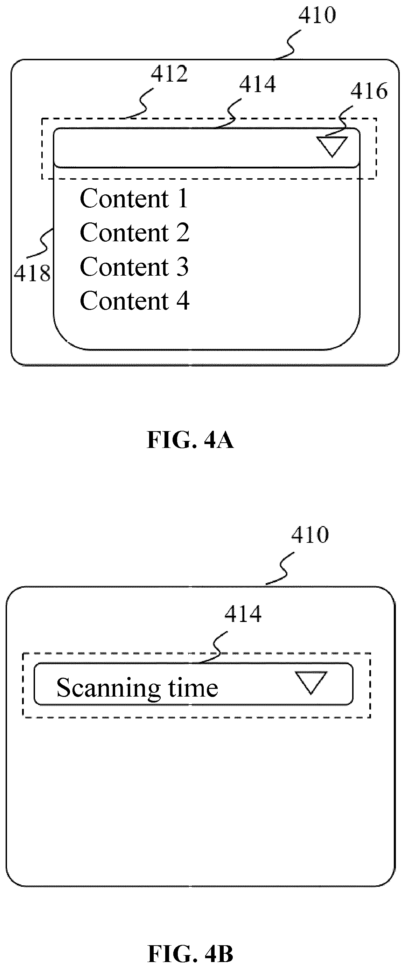

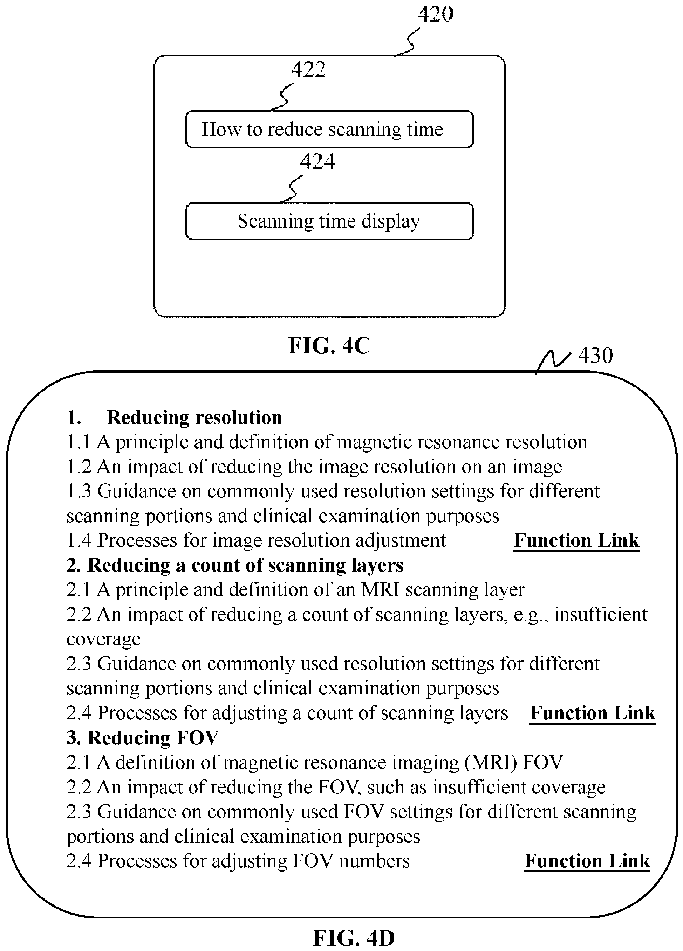

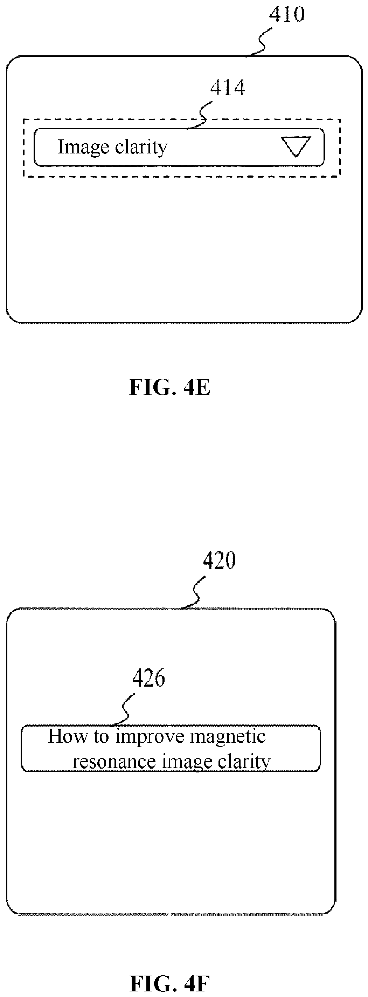

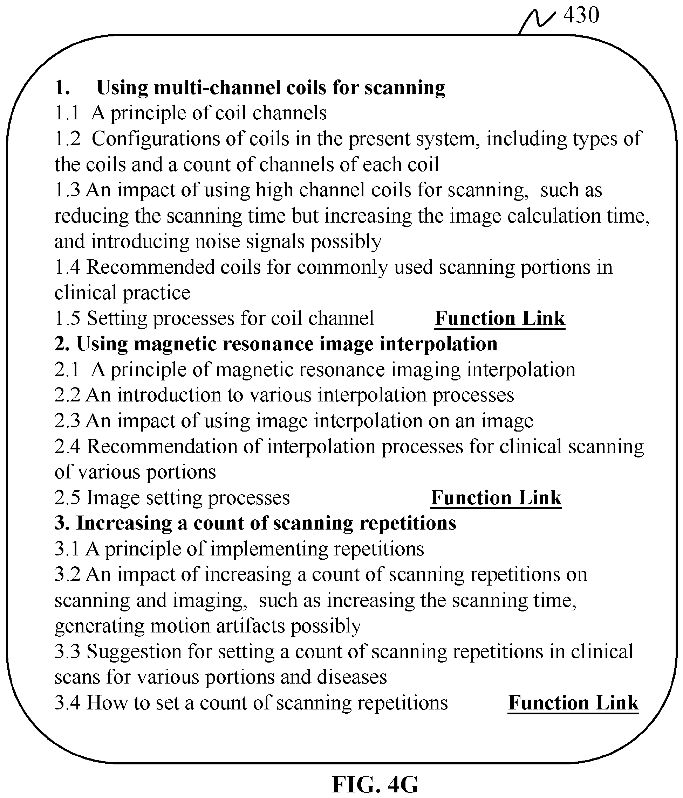

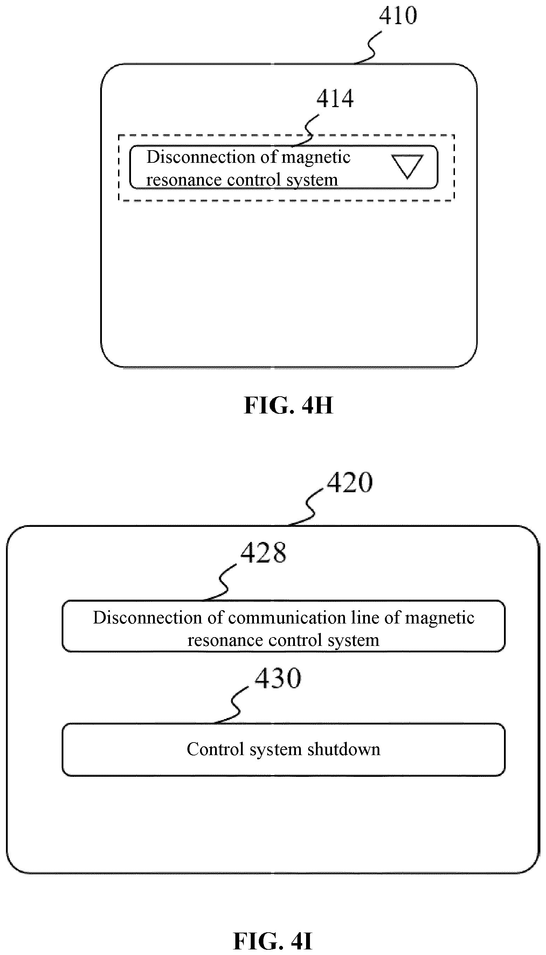

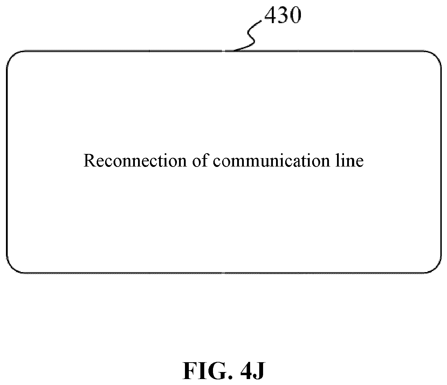

More descriptions of various modules in the system 200 may be found in flowchart in the present disclosure, such as FIG. 4 and related descriptions.

FIG. 3 is a flowchart illustrating an exemplary process for guiding an operation of a medical device according to some embodiments of the present disclosure. In some embodiments, a process 300 may be executed by the processor 110. As shown in FIG. 3, the process 300 may include the following operations:

In 310, retrieving information input by a user is obtained. In some embodiments, operation 310 is performed by the obtaining module 210.

The retrieving information refers to information configured to query or obtain required information and/or data. The retrieving information includes a retrieving keyword, a related phrase, or the like. For example, the retrieving information includes "how to improve MRI image quality" and "image clarity".

In some embodiments, the retrieving information includes a corresponding target task. The target task refers to a relevant task that the user needs to understand. The target task includes at least one of a general knowledge retrieving task, an image acquisition task, and a fault detection task.

The general knowledge retrieving task refers to retrieval of extensive and fundamental principle knowledges related to the medical device. The general knowledge retrieving task refer to a task of understanding basic information such as a model, a system composition, and a principle of the medical device. For example, the user may input retrieving information related to the general knowledge retrieving task to understand relevant information of the corresponding medical device. The general knowledge retrieving task may be applied to a popularization of the medical device and basic learning scenarios. For example, the general knowledge retrieving task may be applied in medical teaching classrooms or for inexperienced users to conduct a basic learning for the medical device.

The image acquisition task refers to a task related to obtaining the medical image based on the medical device. For example, the image acquisition task may be a brain magnetic resonance imaging, a head magnetic resonance angiography, or other tasks based on the medical device to capture the medical image. For example, when the retrieving information includes "how to improve MRI image quality," a target task corresponding to the retrieving information is to improve the image quality, which belongs to the image acquisition task.

The fault detection task refers to a task related to troubleshooting, testing, and maintenance of the medical device and/or system. The fault detection tasks may include but are not limited to a fault localization, a fault type determination, a fault recovery of the medical device, or the like. The fault includes but is not limited to a loss of connectivity in the control system of the medical device, an abnormal image reconstruction, an inadequate image scanning signal strength, or the like.

In some embodiments, the retrieving information includes multiple forms. The retrieving information includes at least one of text information, voice information, and image information related to the target task. For example, the retrieving information includes text information of "how to improve a resolution of MRI images", and the retrieving information includes an MRI image obtained by the medical device currently. As another example, the retrieving information includes voice information such as "medical device abnormality" or "medical device unresponsive" input by the user.

In some embodiments, the user may input the retrieving information in the apparatus 150 through various manners. For example, the user may input the retrieving information through text input, voice input, image input, and other processes. As another example, the user may also select the retrieving information from commonly used retrieving information.

The processor 110 may communicate with the apparatus 150 and read the retrieving information input by the user in the apparatus 150.

As shown in FIG. 4A, the user may input the retrieving information by performing various operations in an information retrieving interface 410 displayed on the apparatus 150. The information retrieving interface 410 may include an operating configuration option 412 for inputting the retrieving information, i.e., providing a configuration for the user to choose an input process. The operating configuration option 412 includes a commonly used retrieving information option 418 and/or retrieving information input box 414. The commonly used retrieving information option 418 may be obtained by operating a drop-down control 416. The user may input the retrieving information by one manner of the operating configuration option 412 (e.g., the retrieving information input box 414/commonly used retrieving information option 418). The user may operate the drop-down control 416 to obtain the commonly used search information option 418, and select desired retrieving information from the retrieving information option 418. The user may input the retrieving information in the retrieving information input box 414, such as text information. As shown in FIG. 4B, the user may input "scanning time: in the retrieving information input box 414 of the information retrieving interface 410 to obtain two retrieving options related to the "scanning time," such as "how to reduce the scanning time" and "scanning time display," displayed in the first information feedback interface 420 shown in FIG. 4C, and the user may select "how to reduce the scanning time" and/or "scanning time display" to view corresponding guide information.

In some embodiments, the information retrieving interface may be embedded in a form of a program or process in the apparatus 150 (e.g., a mobile phone, a tablet, a laptop, etc.), and communicated with a voice input module, an image input module in the apparatus 150. The user may input the retrieving information through a built-in voice input module and image input module of the apparatus 150. For example, the user may also open the information retrieving interface through the apparatus 150, and then input the retrieving information in the form of voice through a microphone. As another example, the user may also access the storage device (e.g., an album) of the apparatus 150 through the information retrieving interface, and select an image related to the retrieving information to input the retrieving information in a form of the image.

In 320, based on the retrieving information, at least one piece of guide information corresponding to the retrieving information is determined. In some embodiments, operation 320 is performed by processing module 220.

The guide information refers to information used to assist and guide the user in completing the target task. The guide information includes at least one of an operation instruction, an operation process, an operation guideline, an operation prompt, an operation demonstration, or the like. A display form of the guide information may be various display forms such as a picture, a text, a video, or any combination thereof. The guide information further includes a working principle of relevant operations for completing the target task, specific function links and/or impact of the operation process, the image, and the systems related to the target task. The user may jump to a target operating interface of the target task by clicking on the function link. In some embodiments, the guide information may also be referred to as operation guide information, and the target operating interface may also be referred to as a functional configuration interface.

In some embodiments, the user may input information related to the medical device and/or system fault in the retrieving information input box of the information retrieving interface through the apparatus 150 to obtain at least one piece of guide information related to the fault. The user may input the retrieving information through the information retrieving interface on the apparatus 150, and select a retrieving option corresponding to the retrieving information on the first information feedback interface to obtain the at least one piece of guide information displayed on the second information feedback interface of the apparatus 150.

As shown in FIG. 4B, the user may input the "scanning time" in the retrieving information input box 414 of the information retrieving interface 410 of the apparatus 150, the apparatus 150 may display the first information feedback interface 420 as shown in FIG. 4C, which includes a retrieving option related to image clarity, including a control 422 corresponding to "how to reduce the scanning time" and a control 424 corresponding to "scanning time display." In response to the user operation, the processor 110 may display corresponding operation guide information in the second information feedback interface 430 of the apparatus 150. When the user triggers the control 424, the operating guide information shown in FIG. 4D including operation guide information related to reducing a resolution, reducing a scanning layer, and reducing a field of view (FOV) may be obtained. The operation guide information related to reducing the resolution includes a principle and definition of magnetic resonance resolution, an impact of reducing the image resolution on the image, such as reducing the image clarity, guidance on commonly used resolution settings for different scanning body parts and clinical examination purposes, processes for image resolution adjustment, function link or the like. The operation guide information related to reducing the scanning layer includes a principle and definition of a MRI scanning layer, an impact of reducing the count of scanning layers, such as insufficient coverage, guidance on commonly used resolution settings for different scanning body parts and clinical examination purposes, processes for adjusting the count of scanning layers, and function links, or the like. The operation guide information related to reducing the FOV includes a definition of magnetic resonance imaging (MRI) FOV and an impact of reducing the FOV, such as insufficient coverage, guidance on commonly used FOV settings for different scanning body parts and clinical examination purposes, processes for adjusting FOV numbers, function links, or the like.

As shown in FIG. 4E, the user may also input "image clarity" in the retrieving information input box 414 of the information retrieving interface 410 of the apparatus 150, the apparatus 150 may display the first information feedback interface 420 as shown in FIG. 4F, which includes retrieving options related to image clarity including a control 426 corresponding to "how to improve magnetic resonance image clarity". Further, the user may trigger the control 426, and the apparatus 150 may display the second feedback information interface 430 as shown in FIG. 4G. The second feedback information interface 430 includes multiple guide information corresponding to "image clarity". The specific guide information includes: using multi-channel coils for scanning, using magnetic resonance image interpolation, and increasing the count of scanning repetitions; wherein using the multi-channel coils for scanning includes a principle of coil channels, a configuration of the coils in the system, including types, a count of channels for each coil, and an impact of using high channel coils for scanning, such as reducing the scanning time but increasing the image calculation time, operating guide information such as a possibility of introducing certain noise signals, recommending commonly used clinical coil, setting processes for coil channel, and corresponding function links for operation guide information, or the like may be introduced. Using magnetic resonance image interpolation includes a principle of magnetic resonance imaging interpolation, an introduction to various interpolation processes, and an impact of using image interpolation on the image, such as operating guide information such as increasing the image calculation time without increasing the scanning time, recommending the interpolation processes for clinical scanning of various portions, image setting processes, corresponding function links, or the like. Increasing the count of scanning repetitions includes a principle of implementing repetition frequency, an impact of increasing repetition frequency on scanning and imaging, such as operating guide information such as increasing the scanning time, generating motion artifacts possibly, suggestion for setting the count of repetitions of clinical scans for various portions and diseases, or the like.

As shown in FIG. 4H, the user may also input "disconnection of a magnetic resonance control system" in the retrieving information input box 414 of the information retrieving interface 410 through the apparatus 150, a retrieving option corresponding to the retrieving information as shown in FIG. 4I in the first information feedback interface 420, i.e., "disconnection of a communication line of the magnetic resonance control system" and a corresponding control 428 and "control system shutdown" and a corresponding control 430 may be obtained. Further, the user may obtain a second feedback information interface 430 as shown in FIG. 4J by triggering the control 428, which may include guide information corresponding to "disconnection of a magnetic resonance control system". More specific, the retrieving option corresponding to the retrieving information includes suggestion for troubleshooting the disconnection of the magnetic resonance control system, i.e., reconnecting a communication line.

In some embodiments, the processor 110 may determine, based on the retrieving information, at least one piece of guide information corresponding to the retrieving information. The processor 110 may determine a matching degree between the retrieving information and multiple reference guide information in the medical device knowledge base, and determine at least one piece of guide information of the multiple reference guide information based on the matching degree. For example, the processor 110 may perform a semantic analysis on the retrieving information and the multiple reference guide information in the medical device knowledge base to determine a semantic matching degree between the retrieving information and the multiple reference guide information, and determine reference guide information that the semantic matching degree satisfying a preset condition (e.g., being greater than a preset matching threshold) as the at least one piece of guide information. In some embodiments, the medical device knowledge base includes a general region and a dedicated region, wherein the general region is configured to store reference guide information for medical devices with multiple models, and the dedicated region is configured to store specific reference guide information for medical devices with different models. Each dedicated region corresponds to a model of the medical device respectively. The dedicated region includes multiple medical device numbers, manual operation guide information corresponding to each number, and task description information corresponding to each manual operation guide information. The medical device number refers to a model of the medical device. In some embodiments, the processor 110 may obtain a dedicated region of the medical device knowledge base corresponding to the model of the medical device, and retrieving in the general region and/or the dedicated region of the medical device knowledge base based on the keyword to determine the at least one piece of guide information.

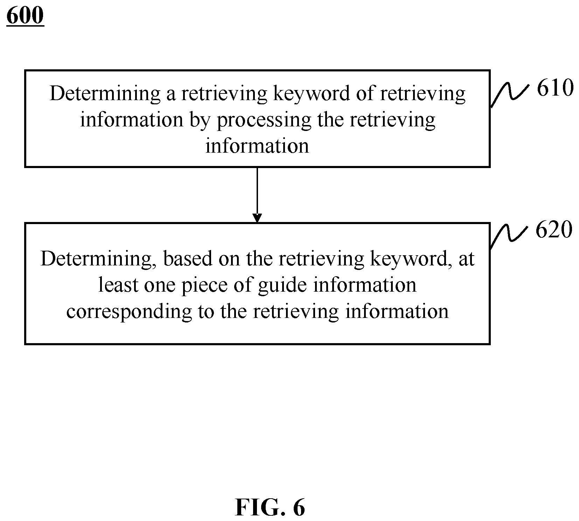

In some embodiments, when the retrieving information includes the text information and/or the voice information, the processor 110 may determine a retrieving keyword of the retrieving information by processing the retrieving information; determine, based on the retrieving keyword, the at least one piece of guide information corresponding to the retrieving information. More descriptions of determining the guide information based on the retrieving keyword may be found in FIG. 6 and related descriptions.

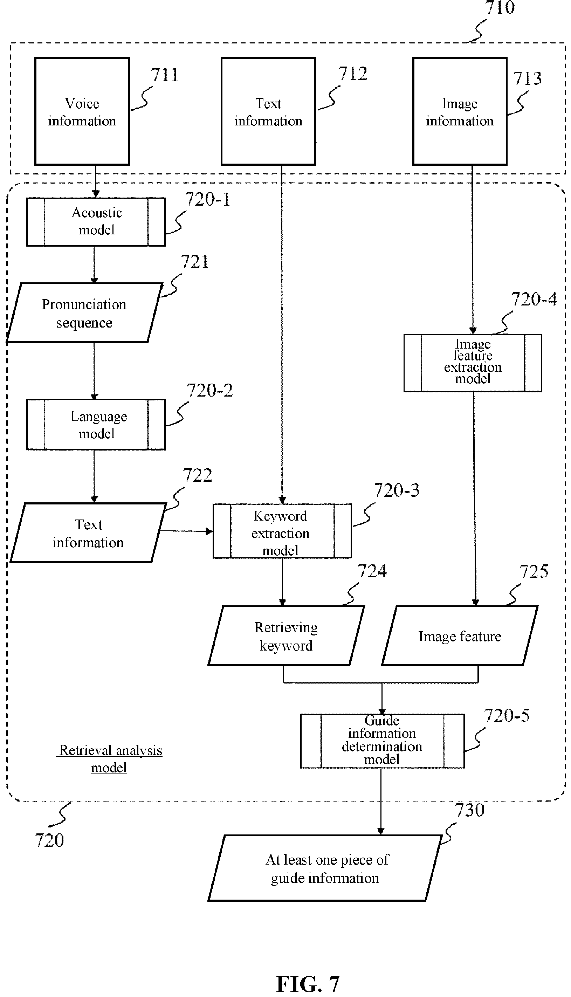

In some embodiments, the processor 110 may determine the at least one piece of guide information corresponding to the retrieving information by processing, based on a retrieval analysis model, the retrieving information, wherein the retrieval analysis model is a machine learning model. More descriptions of determining the at least one piece of guide information based on the retrieval analysis model may be found in FIG. 7 and related descriptions.

In some embodiments of the present disclosure, the guide information corresponding to the target task may be determined based on the retrieving information or the retrieving keyword in the retrieving information. Through the above process, the user may obtain corresponding guide information from the medical device knowledge base based on usage requirements to assist in relevant operations. The process of obtaining the guide information is convenient and fast, making it easier for the user to become familiar with the medical device more quickly. At the same time, the guide information may be displayed and guided in various forms, making it more intuitive and understandable, improving the convenience of using the medical device, making the use of the medical device more efficient, and improving the processing efficiency of completing the target task.

In some embodiments, considering a potential incompleteness of the operating guide information, when the processor 110 fails to find matching guide information in the medical device knowledge base based on retrieving information, feedback information of the target task may be generated. The feedback information is configured to indicate that the operating information database lacks manual operating guide information related to the target task. The processor 110 may report the retrieving information to a service provider of the medical device through the apparatus 150, in order to obtain corresponding operation guide information from the service provider. For example, the corresponding retrieving information may be reported to a customer service personnel of the service provider, or directly uploaded to a service system of the service provider through the medical device system. The service provider may provide the corresponding operation guide information based on the uploaded retrieving information.

In some embodiments, the guide information further includes a customer service link. The customer service link is configured to establish a user dialogue window with a customer service end of the medical device. In some embodiments, the user dialogue window may be configured in the apparatus or in an operation terminal of the medical device. It should be understood that the user dialogue window on the customer service end provides the user with customer service functions related to the medical device, and the user dialogue window can achieve voice robot and/or manual customer service.

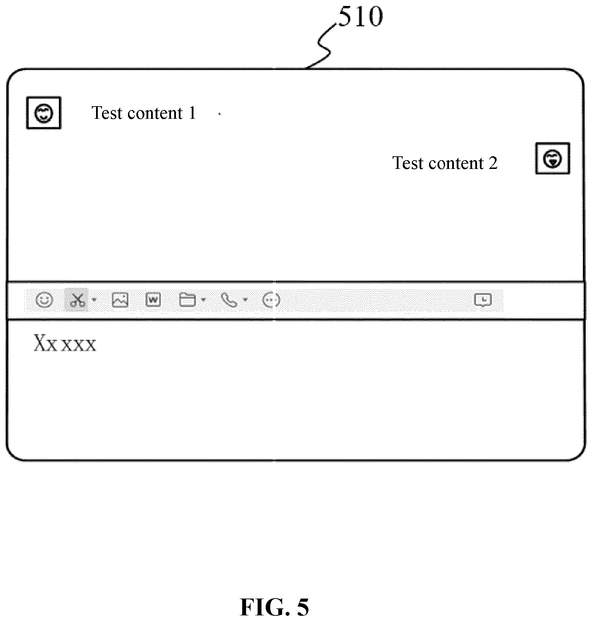

When the user is unable to obtain the operating guide information corresponding to the retrieving information, or needs to further understand other relevant information of the target task or other tasks, a user dialogue window may be established with the customer service end of the medical imaging device by triggering the customer service link. More descriptions may be referred to be as the user dialogue window 510 shown in FIG. 5.

The user may establish a user dialogue window with the customer service end of the medical device through the client, which facilitates the user to obtain operating guide information related to the target task through the user dialogue window when the guide information cannot be obtained based on the retrieving information.

In some embodiments, when the at least one piece of guide information includes multiple pieces of guide information, the processor 110 obtains at least one of a historical operation record of the user, user information, and a current scanning body part.

The historical operation record refers to a historical record of the user operation on the current medical device. For example, the historical operation record includes but is not limited to operation types, operation proficiency, operation standards, historical retrieving information, historical guide information clicked by the user, or the like.

The user information refers to information related to operations of the medical device by the user. For example, the user information includes user identity information, an operation experience, an operation error rate, or the like.

The current scanning body part refers to a body portion of a patient that needs to be scanned corresponding to the target task, wherein the target task is a task that the user performs currently. For example, the current scanning body part includes a head and neck, an abdomen, a chest, or the like.

The processor 110 may obtain the historical operation record of the user, the user information, and the current scanning body part of the patient through various processes. For example, the user information and the corresponding historical operation record may be associated and stored in medical device 160. After obtaining the user permission, the processor 110 may obtain the user information stored in the medical device 160 and the corresponding historical operation record. For example, the user information and the corresponding historical operation record may be associated and stored in the medical device 160. After obtaining the user permission, the processor 110 may obtain the user information stored in the medical device 160 and the corresponding historical operation record. As another example, each time the user operates the medical device 160, the user information may be entered manually or through a facial identification, and the operation record may be synchronously associated and stored with the user information during each operation. The processor 110 may obtain the user information through the facial identification, and determine an associated historical operation record based on the user information. As another example, the processor 110 may obtain the current scanning body part input by the user through the apparatus 150.

In some embodiments, the processor 110 determines a sequence number for each of the multiple pieces of guide information based on the multiple pieces of guide information and the at least one of the historical operation record, the user information, and the current scanning body part.

In some embodiments, the processor 100 may determine a sequence number for each of the multiple pieces of guide information based on the multiple pieces of guide information and the at least one of the historical operation record, the user information, and the current scanning body part by a sequencing model, wherein the sequencing model is a machine learning model. The sequencing model may be a convolutional neural networks (CNN), a deep neural networks (DNN), or at least one of other custom models.

In some embodiments, the sequencing model is obtained by training. In some embodiments, a first training sample of the sequencing model includes a sample historical operation record, sample user information, sample scanning body part, and multiple pieces of sample guide information corresponding to sample retrieving information. The first training label may be an actual sorting result of the multiple sample guide information in the first training sample, and the first training label may be determined by professional personnel (e.g., equipment vendors) to evaluate the multiple historical guide information. In some embodiments, the processor 110 may input the first training sample into an initial sequencing model and construct a loss function based on an output of the initial sequencing model and the first training label. Based on the loss function, parameters of the initial sequencing model may be updated through a gradient descent process or other processes until satisfying a preset condition, and the training is completed to obtain a trained sequencing model. The preset condition may be a convergence of the loss function or the training reaching a maximum number of training iterations.

In some embodiments of the present disclosure, the multiple pieces of guide information may be sequenced based on at least one of the historical operation record, the user information, and the current scanning body part, a suitable guide information may be determined based on the sequence result. When determining the guide information in above process, a user experience of the user, an error rate, a historical common operation, and a current target task are considered, which make the determined guide information more in line with the user requirements and more relevant to current usage scenarios.

FIG. 6 is a flowchart illustrating an exemplary process for determining at least one piece of guide information according to some embodiments of the present disclosure. In some embodiments, the process 600 is performed by the processor 110.

As shown in FIG. 6, when the retrieving information includes the text information and/or the voice information, the processor 110 may perform the process 600 for determining the at least one piece of guide information corresponding to the retrieving information. The process 600 may include the following operations:

In 610, a retrieving keyword of the retrieving information may be determined by processing the retrieving information. In some embodiments, the operation 610 is performed by the processing module 220.

The retrieving keyword refers to a keyword used in the retrieving information to determine the guide information. A piece of retrieving information includes one or more retrieving keywords, and the part of speech of the multiple retrieving keyword may be the same or different. For example, when the retrieving information is "how to improve MRI image resolution", the retrieving keyword in the search information may include "improve", "MRI image", and "resolution", wherein "improve" is a verb, "MRI image" and "resolution" are nouns.

In some embodiments, the processor 110 may determine the retrieving keyword from the retrieving information through various processes. In some embodiments, the processor 110 may process the retrieving information through a keyword extraction model to determine the retrieving keyword. Parameters of the keyword extraction model may be determined based on the key extraction model in a trained retrieval analysis model, more descriptions of the keyword extraction model may be found in FIG. 7 and related descriptions.

In some embodiments, the processor 110 may obtain multiple reference keywords. The reference keyword refers to a keyword used for reference, which may be set in various ways. For example, the processor 110 may extract the keyword from the historical operation record (e.g., the keyword extracted based on a Word2Vec word vector, a K-means clustering algorithm, etc.), obtain multiple candidate keywords, and count the number of times each candidate keyword appears. As another example, the processor 110 may extract the keyword related to the medical device from the medical device knowledge base, obtain the multiple candidate keywords, and count the number of times each candidate keyword appears. In response to the number of times a candidate keyword appears exceeding a preset threshold, the candidate keyword may be determined as a reference keyword.

In some embodiments, the processor 110 may extract the keyword from the retrieving information to determine at least one keyword to be identified in the retrieving information.

In some embodiments, for each keyword to be identified, the processor 110 may determine a similarity degree between the keyword to be identified and each of the multiple reference keywords. The process of determining the keyword to be identified and the multiple reference keywords may be performed in various ways, for example, a similarity degree between the keyword to be identified and the reference keyword may be determined based on a vector distance between feature vectors corresponding to the keyword to be identified and the reference keyword.

When the similarity degree between the keyword to be identified and the reference keyword may be determined satisfies a preset similarity condition, the processor 110 may determine the keyword to be identified as the retrieving keyword. The preset similarity condition includes the similarity degree between the keyword to be identified and the reference keyword being greater than or equal to a first similarity threshold (e.g., 100%).

In some embodiments, the processor 110 may determine the retrieving keyword determined by the keyword to be identified by correcting the keyword to be identified. When the similarity degree between the keyword to be identified and the reference keyword satisfies a preset correction condition, the processor 110 may correct the keyword to be identified, i.e., determining the corresponding reference keyword as a retrieving keyword corresponding to the keyword to be identified. The preset correction condition includes the similarity degree between the keyword to be identified and the reference keyword being greater than a second similarity threshold and being less than the first similarity threshold.

For example, the preset correction condition may be the similarity degree between the keyword to be identified and the reference keyword being greater than 85% and being less than 100%, the keyword to be identified is "MR1 image", and the reference keyword is "MRI image". The processor 110 may determine that the similarity degree between the keyword to be identified and the reference keyword is 92%, in response to the similarity degree satisfies the preset correction condition, the processor may determine the reference keyword "MR1 image" as a retrieving keyword corresponding to the keyword to be identified "MRI image".

In some embodiment of the present disclosure, by correcting the keyword to be identified, the retrieving keyword input by the user may be corrected, which can avoid duplicate input by the user, improve retrieving efficiency, and ensure user experience.

In 620, based on the retrieving keyword, the at least one piece of guide information corresponding to the retrieving information may be determined. In some embodiments, the operation 620 may be performed by the processing module 220.

In some embodiments, the processor 110 may perform modeling or employ various data analysis algorithms, such as regression analysis, discriminant analysis, or the like, to analyze and process the retrieving keyword, and obtain the at least one piece of guide information corresponding to the retrieving information. In some embodiments, the processor 110 may determine the at least one piece of guide information from the medical device knowledge base based on the retrieving keyword using a keyword matching algorithm (e.g., an algorithm such as sequential search, binary search, hash search, etc.). In some embodiments, the processor 110 may determine the at least one piece of guide information from the medical device knowledge base based on a guide information determination model. Parameters of the guide information determination model may be determined based on the guide information in the trained retrieval analysis model, more description of the guide information determination model may be found in FIG. 7 and related descriptions.

The processor 110 may filter out reference guide information corresponding to the retrieving keyword from the medical device knowledge base based on the retrieving keyword, and determine the reference guide information as the guide information. The processor 110 may analyze the retrieving information and determine a task type of the target task corresponding to the retrieving information. When the target task is a general knowledge retrieving task, the processor 110 may determine the reference guide information corresponding to the retrieving keyword from the general region of the medical device knowledge base based on the retrieving keyword corresponding to the retrieving information, and determine the reference guide information as the guide information; when the target task is an image acquisition task or a fault detection task, the processor 110 may determine a dedicated region corresponding to the medical device in the medical device knowledge base based on a first device model used by the user, and determine the reference guide information corresponding to the retrieving keyword from the dedicated region based on the retrieving keyword, and determine the reference guide information as the guide information. the first device model refers to model information of the medical device used by the user.

In some embodiments, the processor 110 may determine at least one piece of guide information from the medical device knowledge base through other manners based on o the retrieving keyword.

The processor 110 may obtain the first device model, first configuration information, and a first principle of the medical device used by the user. The first configuration information refers to relevant configuration information on the medical device used by the user, and the first principle refers to an operating principle of each unit on the medical device used by the user to perform the task. After obtaining the user permission, the processor 110 may obtain the first device model, first configuration information, and the first principle from setting information of the medical device 160.

In some embodiments, the processor 110 may determine the reference information from the medical device knowledge base based on the first device model, the first configuration information, and the first principle. The reference information refers to information in the medical device knowledge base having reference significance for the medical device used by the user. The processor 110 may determine a similarity degree between each candidate reference medical device in the medical device knowledge base and the medical device used by the user based on the first device model, the first configuration information, and the first principle, and determine relevant information corresponding to the reference medical device whose similarity degree exceeds a preset similarity threshold as the reference information.

In some embodiments, the processor 110 may determine a target feature vector corresponding to the medical device by perform the feature extraction on the first device model, the first configuration information, and the first principle. More descriptions of obtaining the target feature vector may be found in following descriptions related to obtaining the reference feature vector. The processor 110 may determine at least one reference feature vector from the at least one candidate reference feature vector in the medical device knowledge base based on the target feature vector. The candidate reference feature vector refers to a feature vector corresponding to the candidate reference medical device in the medical device knowledge base. The candidate reference medical device refers to a medical device corresponding to other medical device information in the medical device knowledge base. The other medical device information includes relevant information other than the medical device used by the user. The reference feature vector refers to a feature vector corresponding to the reference medical device. The processor 110 may determine a distance between the target feature vector and each of at least one candidate reference feature vector, and determine a candidate reference feature vector whose distance is less than a preset distance threshold as the reference feature vector.

In some embodiments, the processor 110 may process a second device model, second configuration information, and a second principle corresponding to the multiple candidate reference devices based on a target embedding layer to determine each corresponding candidate reference feature vector among the multiple candidate reference medical devices. The second device model refers to model information of the candidate reference medical device. The second configuration information refers to relevant configuration information of the candidate reference medical device, and the second principle refers to an operating principle of each unit on the candidate reference medical device to perform the task.

In some embodiments, the processor 110 may train a vector similarity degree determination model to obtain the target embedding layer. The vector similarity degree determination model includes a first embedding layer, a second embedding layer, and a similarity degree judgment layer. The processor 110 may input the second device model, the second configuration information, and the second principle corresponding to two candidate reference medical devices into the first and second embedding layers, respectively. An output of the first and second embedding layers includes candidate reference feature vectors corresponding to the two candidate reference medical devices. An input of the similarity degree judgment layer includes candidate reference feature vectors corresponding to two candidate reference medical devices, and an output includes a similarity degree of feature vectors corresponding to the two candidate reference medical devices.

In some embodiments, the processor 110 may train the vector similarity degree determination model. The processor 110 may obtain multiple sets of labeled second training samples, wherein the second training sample includes sample device model information, sample configuration information, and sample principles of two sample devices. The second training label includes a sample similarity degree between the feature vectors corresponding to the two sample devices. The second training sample may be obtained based on the medical device knowledge base, and the second training label may be determined by manually evaluating the two sample devices.

For the each set of second training sample with labels, the processor 110 may input the sample device model information, the sample configuration information, and the sample principle of a set of sample devices in the second training sample into an initial first embedding layer in an initial vector similarity degree determination model, input the sample device model information, the sample configuration information, and the sample principle of another set of sample devices in the second training sample into an initial second embedding layer in an initial vector similarity degree determination model, and input outputs of the initial first embedding layer and the initial second embedding layer into an initial similarity degree judgment layer in the initial vector similarity degree determination model. Based on the output of the initial similarity judgment layer and the corresponding second training label of the second training sample, a loss function is constructed, and the parameters of the initial first embedding layer, initial second embedding layer, and initial similarity judgment layer are updated based on the loss function, when a preset training condition is satisfied, a trained vector similarity degree determination model is obtained. The preset training condition includes but is not limited to convergence of the loss function, reaching a threshold for a training period, or the like.

In some embodiments, the processor 110 may determine the first embedding layer or second embedding layer in the vector similarity determination model as a target embedding layer.

In some embodiments of the present disclosure, by training the overall vector similarity determination model, the target embedding layer is obtained, which can avoid the problem of difficulty in obtaining the training labels when training the target embedding layer separately.

In some embodiments, the processor 110 may determine a reference device corresponding to the at least one reference feature vector, and determine data information corresponding to the medical device and the reference device in the medical device knowledge base as the reference information. The processor 110 may determine the at least one piece of guide information from the reference information in various ways based on the retrieving keyword. For example, the processor 110 may determine the at least one piece of guide information from reference information through the keyword matching algorithm based on the retrieving keyword. As another example, the processor 110 may also determine the at least one piece of guide information from the reference information by determining the guide information determination model based on the retrieving keyword.

By extracting the keyword in the retrieving information in some embodiments of the present disclosure, the corresponding guide information can be determined quickly and efficiently, and the using experience of the medical device can be improved.

FIG.7 is a schematic diagram illustrating an exemplary retrieval analysis model according to some embodiments of the present disclosure.

As shown in FIG. 7, the processor 110 may determine at least one piece of guide information 730 corresponding to the retrieving information by processing retrieving information 710 based on the retrieval analysis model 720. The retrieval analysis model is a machine learning model. In some embodiments, the processor 110 may determine an internal structure of the retrieval analysis model based on types of retrieving information. The retrieval analysis model 720 includes a guide information determination model 720-5.

When the retrieving information 710 includes text information 712, the processor 110 may determine that the retrieval analysis model 720 also includes a keyword extraction model 720-3. The keyword extraction model 720-3 may extract a retrieving keyword from the text information. An input of the keyword extraction model 720-3 includes text information 712 in the retrieving information 710, and includes text information 722 determined based on voice information 711. An output of the keyword extraction model 720-3 includes a retrieving keyword 724. The keyword extraction model 720-3 may be a deep learning model or other machine learning models that can achieve the function.

When the retrieving information 710 includes the voice information 711, the processor 110 may determine the retrieval analysis model 710 further including an acoustic model 720-1, a language model 720-2, and a keyword extraction model 720-3. The acoustic model 720-1 may be configured to extract a pronunciation sequence of basic pronunciation in a chronological order from the voice information 711. The language model 720-2 may be configured to obtain multiple words by sequentially combining pronunciation sequences in order, and perform semantic analysis on the multiple words in a directional semantic order to obtain the text information 722. An input of the acoustic model 720-1 may be the voice information 711, and an output of the acoustic model 720-1 may be a pronunciation sequence 721. An input of the language model 720-2 includes a pronunciation sequence 721, and an output includes textual information 722. The acoustic model may be an acoustic model composed of a Hidden Markov Model (HMM)+Gaussian Mixture Model (GMM) or an acoustic model composed of HMM+Deep Neural Network (DNN). The language model may be a N-Gram language model, a Chinese Language Models (CLM), or the like. In some embodiments, when the retrieving information 710 includes voice information 711, and the retrieval analysis model 710 includes a denoising layer (not shown in the figure). In some embodiments, the voice information may be referred to as voice instructions. The denoising layer may be configured to filter and denoise voice instructions, determine audio data with no noise. The processor 110 may input the audio data with no noise into an acoustic model 720-1. In a process of inputting the voice instructions, in addition to human voice, there may also be many other audio impurities, such as environmental noise, echo, reverberation, or the like, so it is necessary to filter and reduce the noise; a least Mean Square (LMS) adaptive filtering, a Wiener filtering, and other manners may be configured to eliminate the environmental noise, the echo, the reverberation, and other noise in the voice instructions; the audio data refers to sound data that retains strongest, most regular, longest lasting signal, and sound characteristics closest to human voice characteristics after eliminating the noise from the voice instructions.

When the retrieving information 710 includes image information 713, the processor 110 may determine the retrieval analysis model 720 including an image feature extraction model 720-4. The image feature extraction model 720-4 may be configured to extract image features from the image information 713. An input of the image feature extraction model 720-4 includes image information 713, and an output of the image feature extraction model 720-4 includes an image feature 725. The image feature extraction model 720-4 may be a convolutional neural network model or other machine learning models that can achieve the function.

An input of the guide information determination model 720-5 includes keyword 724 and/or image features 725, and the output includes at least one piece of guide information 730. The guide information may determine the model 720-5 being a deep learning model or other machine learning models that can implement corresponding functions. An input of the guide information determination model 720-5 includes retrieving keyword 724 and/or image features 725, and the output of the guide information determination model 720-5 includes at least one guide information 730. The guide information may determine the model 720-5 being a deep learning model or other machine learning models that can implement corresponding functions.

In some embodiments, the processor 110 may obtain a retrieval analysis model 720 through training. The third training sample includes sample retrieving information, and the third training label includes at least one piece of guide information in the medical device knowledge base. The third training sample may be obtained based on the historical retrieving information corresponding to the medical device, and the third training label may be manually annotated based on the sample retrieving information. The processor 110 may input the third training sample into an initial retrieval analysis model, construct a loss function based on the output of the initial retrieval analysis model and the third training label, and iteratively update the parameters of the initial retrieval analysis model based on the loss function, when satisfying a preset condition, the training is ended, and a trained retrieval analysis model is obtained. The preset condition includes but is not limited to convergence of the loss function, training period reaching a threshold, or the like.

In some embodiments of the present disclosure, by processing the retrieving information based on the retrieval analysis model, which can determine at least one piece of guide information corresponding to the retrieving information quickly and accurately, assist the user in familiarizing themselves with the operation of medical device, and improve operational efficiency.

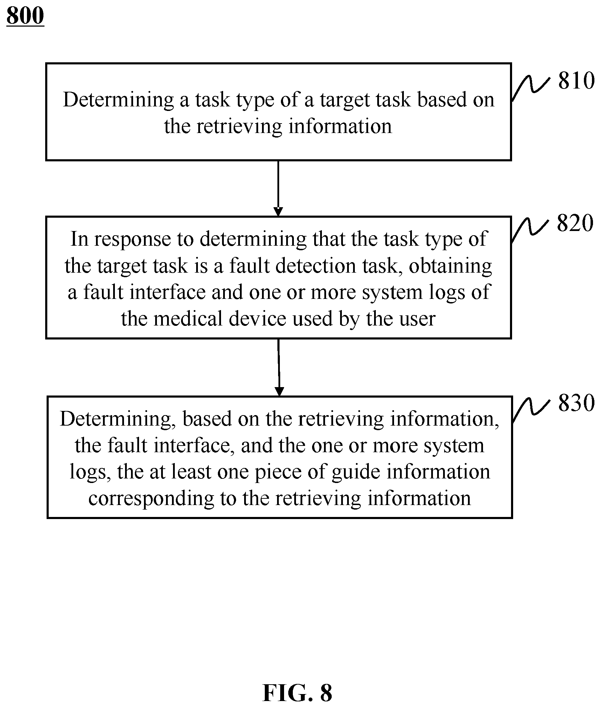

FIG. 8 is a flowchart illustrating another exemplary process for determining at least one piece of guide information according to some embodiments of the present disclosure. In some embodiments, the process 800 may be performed by the processor 110. As shown in FIG. 8, the process 800 may include the following operations:

In 810, a task type of the target task may be determined based on the retrieving information. In some embodiments, the operation 810 may be performed by the processing module 220.

In some embodiments, the processor 110 may determine the retrieving keyword in the retrieving information based on the retrieving information; and determine the task type of the target task based on the retrieving keyword. More descriptions of determining the retrieving keyword may be found in FIG. 6 and related descriptions. In some embodiments, the processor 110 may match a retrieving keyword with at least one keyword in a key word library corresponding to the multiple task types of the target task, determine a keyword that satisfies a preset condition, and determine a corresponding type task of the target task based on the keyword. The preset condition includes a matching degree being greater than a matching degree threshold. The matching degree threshold may be manually set or set by the system default. The matching manner includes but is not limited to semantic matching, vector matching, or the like.

In 820, in response to determining that the task type of the target task is the fault detection task, a fault interface and one or more system logs of the medical device used by the user may be obtained. In some embodiments, operation 820 may be performed by the processing module 220.

The fault interface refers to an interface used to display information related to a control system fault of the medical device. For example, the fault interface may be configured to the display information related to a fault such as a disconnection of the communication line and control system shutdown of the medical device, including a fault code, troubleshooting suggestion, or the like.

The system log refers to information recorded in the control system of the medical device regarding hardware, software, and system issues. The system log may also record events that occurred in the system. The processor 110 may determine cause of faults and/or errors through the system log.

In some embodiments, when the task type of the target task is a fault detection task, the processor 110 may send instructions to the medical device 160 to obtain a fault interface of a last occurrence of the fault in the medical device 160. The processor 110 may further determine a time period during which the failure occurred and obtain the system log during the time period.

In 830, the at least one piece of guide information corresponding to the retrieving information may be determined based on the retrieving information, the fault interface, and the one or more system logs.

In some embodiments, each of the at least one piece of guide information includes a fault type of the medical device and/or troubleshooting suggestion.

The fault type refers to a specific classification of faults, which includes disconnection of the medical device control system circuit, downtime of the medical device control system, the troubleshooting suggestions refers to processes, measures, and operational guidelines used to troubleshoot the fault type. The troubleshooting suggestion includes text, image, animation, and other information that guide the user on how to the troubleshoot. For example, for the disconnection of the medical device control system circuit, the troubleshooting suggestion includes reconnecting the communication line.

In some embodiments, the processor 110 may determine the fault type of the medical device 160 based on the fault interface and the system log using a preset rule. For example, the preset rule includes that if the system log records information including a data communication log, the control system is no longer connected. The processor 110 may determine that the fault type of medical device 160 is a disconnection of the communication line of the medical device control system. As another example, the preset rule includes recording information in the system log as an abnormal operation of the control system component, and the processor 110 may determine that the fault type of medical device 160 is the control system shutdown.

In some embodiments, the processor 110 may determine corresponding troubleshooting suggestion for the fault type from the medical device knowledge base based on the fault type and the retrieving information. For example, for the disconnection of the communication line in a medical device control system, the processor 110 may determine troubleshooting suggestion corresponding to the disconnection of the communication line from the medical device knowledge base in the medical device control system as investigating whether circuits in the medical device control system is disconnected. For example, for the control system shutdown, the processor 110 may determine troubleshooting suggestion for the control system shutdown from the medical device knowledge base as restarting the control system.

In some embodiments, the fault types of medical device 160 may be multiple, and the processor 110 may analyze and process the fault interface and system logs to determine a probability of each fault type, and sort at least one piece of guide information based on the fault probability.

In some embodiments of the preset disclosure, when the medical device occurs fault, the user may be guided to troubleshoot, which can quickly identify the fault type o and guide the user in troubleshooting, improve the efficiency of fault detection and troubleshooting, and ensuring the user experience.

FIG. 9 is a flowchart illustrating another exemplary process for guiding the user in operating on the target operating interface to complete the target task according to some embodiments of the present disclosure. In some embodiments, the process 900 may be performed by the processor 110. As shown in FIG. 9, the process 900 includes the following operations:

In 910, retrieving information input by a user may be obtained, wherein the retrieving information includes a corresponding target task. In some embodiments, the process 910 may be performed by the obtaining module 210.

More description of obtaining the retrieving information input by the user may be found in FIG. 3 and related descriptions.

In 920, based on the retrieving information, at least one piece of guide information corresponding to the retrieving information may be determined. The at least one piece of guide information is configured to guide the user in completing the target task. In some embodiments, the operation 910 may be performed by processing module 220.

More descriptions of determining guide information corresponding to the retrieving information may be found in FIG. 3 and related descriptions.

In 930, in response to a triggering operation of the user, a target operating interface may be determined, and the target operating interface is related to the at least one piece of guide information. In some embodiments, the operation 930 may be performed by the display module 230.

The triggering operation refers to an operation used to trigger the guide information. A display interface on the apparatus 150 may display a target operating interface corresponding to the guide information after triggering. For example, the triggering operation includes user clicking, selecting, and other actions on the guide information on the display interface. As another example, the triggering operation may be the user clicking a function link in the guide information. By clicking on the function link, the display interface in the apparatus 150 may automatically jump to a corresponding target operating interface. By operating in the target operating interface, various parameters related to the target task of the medical device may be set.

The target operating interface refers to an interactive interface where the user operates and controls to complete the target task. In some embodiments, the processor 110 may determine at least one target operating interface based on the guide information. A correspondence between the guide information and target operating interface may be stored in the medical device knowledge base in advance. For example, if the guide information is "how to reduce the scanning time", a specific content of the guide information includes a resolution setting, a scanning layer setting, and a FOV setting. Based on the specific content of the guide information, the target operating interface including a resolution setting interactive interface, a scanning layer setting interactive interface, and a FOV setting interactive interface may be determined.

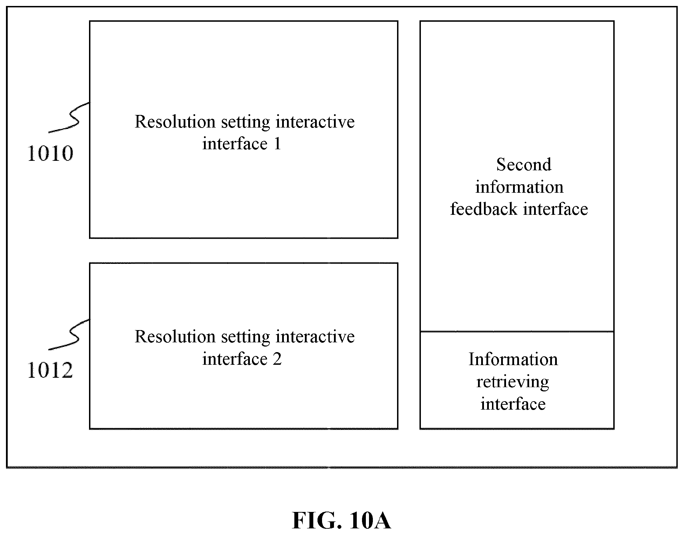

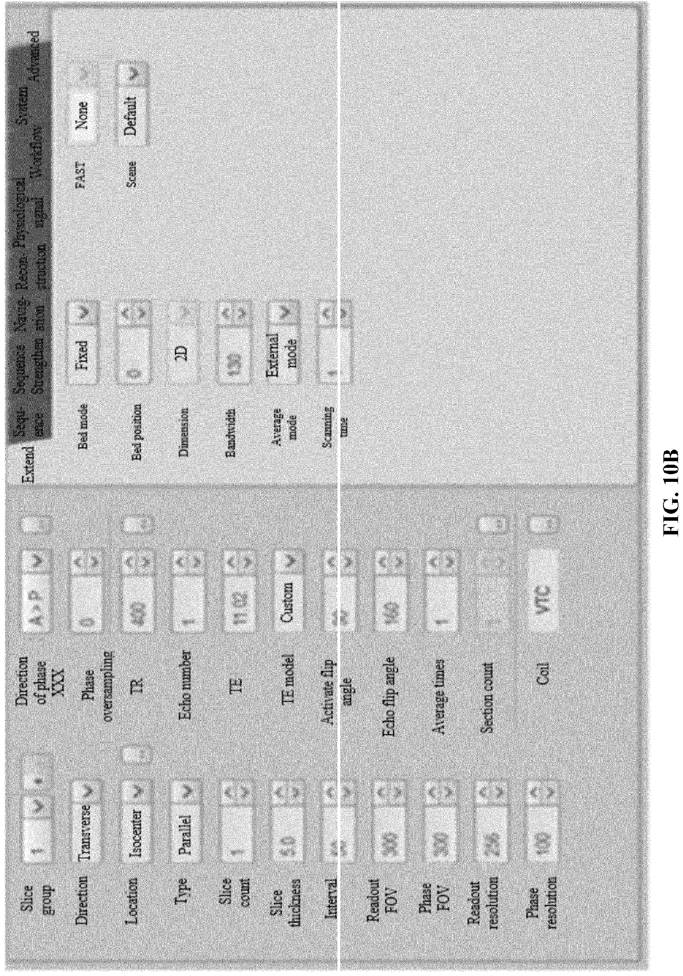

A count of the target operating interface may be one or more. When the user needs to operate on multiple pages to complete the target task, the count of target operating interface may be multiple; when the user needs to operate on one page to complete the target task, the count of the target operating interface may be one. The user may click or select at least one piece of the guide information, and the display interface on the apparatus 150 may display a content related to the guide information and a target operating interface corresponding to the guide information. As shown in FIG. 10A, the target task may be to improve the image resolution. Correspondingly, the target operating interface includes a resolution setting interactive interface 1 (1010) and a resolution setting interactive interface 2 (1012). The user may perform corresponding operations in the resolution setting interactive interface 1 (1010) and the resolution setting interactive interface 2 (1012) based on the specific content corresponding to the guide information displayed in the second information feedback interface to improve the image resolution. For example, the user may enter an interface shown in FIG. 10B by clicking on the resolution setting interactive interface 1 (1010), and adjust the "readout resolution" and "phase resolution" in the interface based on the specific content of the guide information.

In some embodiments, when the user completes different target tasks, in addition to performing settings related to the target task through the target operating interface corresponding to the target task, the user may further directly input operation parameters and other related information related to the target task that need to be set through the information retrieving interface. In some embodiments, the second information feedback interface may also provide feedback to the user on information related to the target task, such as operating parameters, scanning results, or the like. For example, when the target task that the user needs to perform is the image acquisition task, specifically to improve the image quality, the second information feedback interface may provide feedback on relevant parameters set by the user to improve the image quality, so that the user may confirm whether the parameter settings are correct; the second information feedback interface may also provide feedback on an image collected by the image acquisition task, so that the user can check whether the collected images are standardized.

In some embodiments, the user may also click on a function link in the guide information corresponding to the target task, and the display interface of the apparatus 150 may automatically jump to a corresponding target operating interface. For example, when the target task is to improve image resolution, in response to the user clicking on the corresponding function link in the guide information, the display interface of the apparatus 150 may automatically jump to the target operating interface shown in FIG. 10B. It can be seen that the target operating interface includes parameter setting items related to the target task. The user may operate in the target operating interface based on the guide information to complete the parameter setting items r related to the target task.

For example, when the target task is the image acquisition task, the parameter setting item includes any of the setting items for image scanning time, image clarity, and image scanning protocol. When the target task is a fault detection task, the parameter setting item includes any of the setting items for the disconnection of the control system of medical imaging device, abnormal image reconstruction, and a strength of the image scanning signal not satisfying the standard.

More description of relevant parameter setting of performing the image acquisition task and the fault detection task based on the operating guide information may be found in FIG. 4A-FIG. 4J and related descriptions.

In some embodiments, the user may navigate to the target operating interface of the target task through the guide information, allowing them to control on the target operating interface and complete the target task. The direct jump manner can facilitate the user to complete the target task, simplify the operation process, and thus save the operation time.

In some embodiments, in order to facilitate the user in completing the target task, the target operating interface includes operating guide information. The operating guide information is configured to guide the user to complete corresponding operations on the target operating interface. The user may choose whether to display the corresponding operating guide information in the target operating interface. When the user selects displaying the corresponding operating guide information in the target operating interface, the process 900 includes:

In 940, based on the target operating interface and the operating guide information, the user may be guided in operating on the target operating interface to complete the target task. In some embodiments, the operation 940 may be performed by the guide module 240.

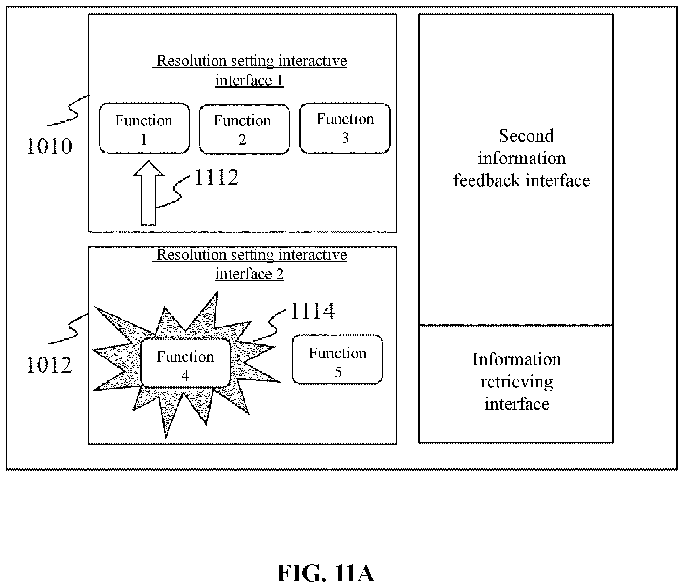

The operating guide information refers to information used to guide the user to perform the operation to complete the target task. The operating guide information include but is not limited to operation processes, operation sequence, operation content, or the like. The processor 110 may enable the user to clarify how to perform operations in the interface. As shown in FIG. 11A, the resolution setting interactive interface 1 (1010) includes the operating guide information, i.e., an arrow 1112, the arrow 1112 may guide the user to perform corresponding operations on "function 1", and the resolution setting interaction interactive 2 (1012) include operation guide information, i.e., a lump of color, the lumps of color may guide the user to perform corresponding operation on "function 2".

In some embodiments, the operating guide information may be displayed through various display types. The display types of operating guide information in the target operating interface include at least one of a text guidance, an image guidance, and an animation guidance. For example, the processor 110 may display the operating guide information through lighting, flashing, different colors, different fonts, rectangular box annotations, or the like, to indicate a next action button that the user needs to operate.

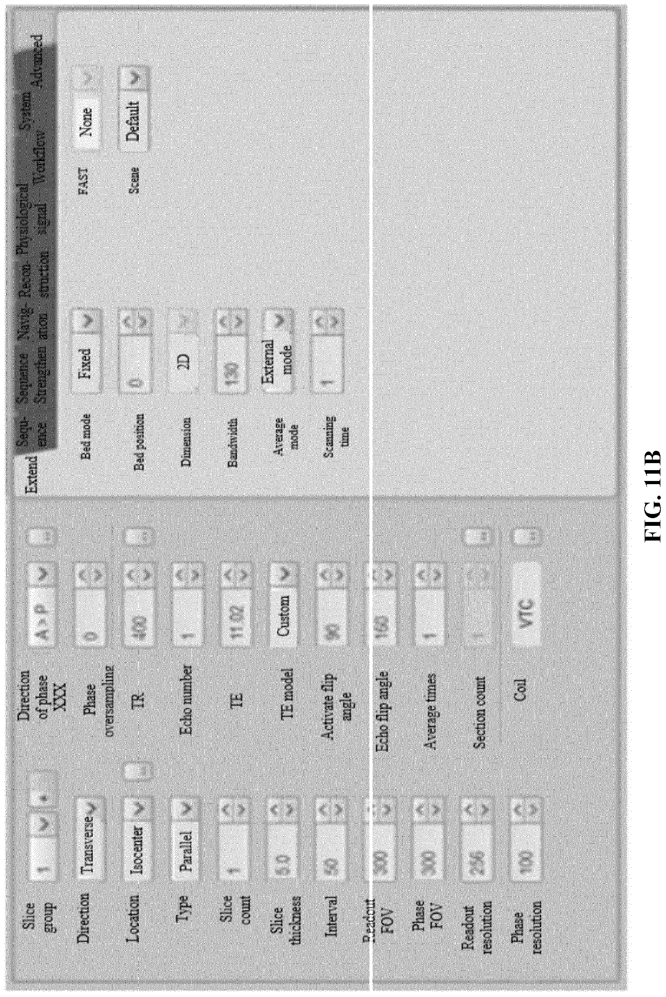

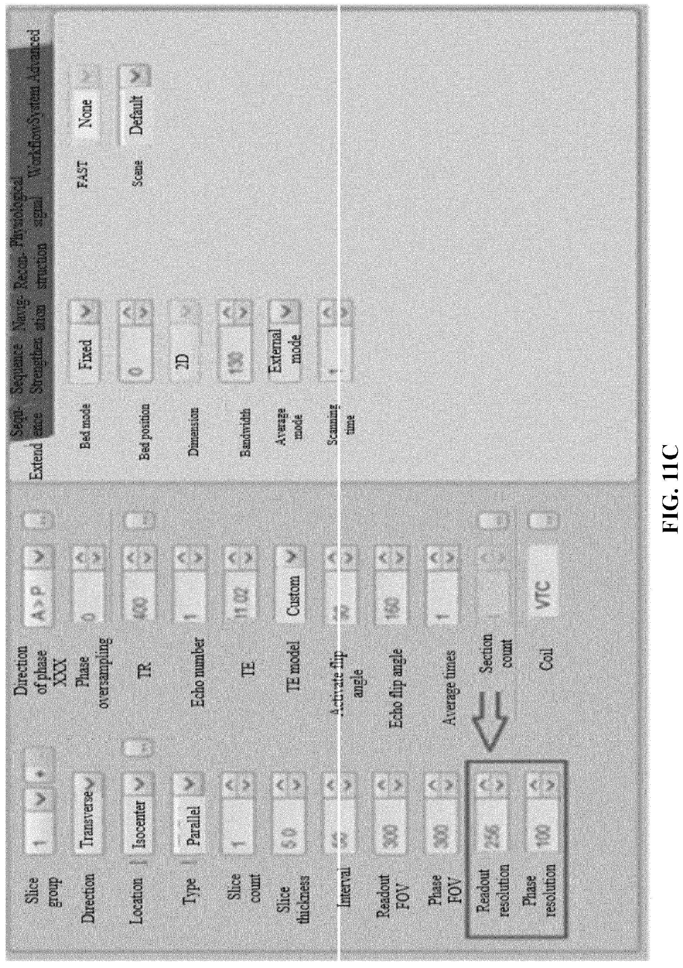

The operating guide information may be used for special display of content that requires execution of operations. The special display may be to highlight, highlight, or differentiate key information (e.g., displaying corresponding text contents in different fonts and colors). As shown in FIG. 11B, the processor 110 may adjust a font corresponding to text descriptions for "readout resolution" and "phase resolution" that require corresponding operations to be performed in the interface to be displayed in gray to distinguish the text descriptions from other functions in the interface. As shown in FIG. 11C, the processor 110 may use rectangular boxes and arrows to highlight the "readout resolution" and "phase resolution" that require corresponding operations in the interface, in order to distinguish the "readout resolution" and "phase resolution" from other functions in the interface.

In some embodiments, for each of the at least one target operating interface, the processor 110 may determine the display type and guidance content of the operating guide information in the target operating interface based on the target operating interface and the guide information.

The processor 110 may analyze and process the guide information, and determine the display type and guidance content of the guide information in the target operating interface based on a preset rule. For example, the processor 110 may analyze and process the guide information to obtain operating contents that needs to be performed in the target operating interface, thereby determining the guide contents, and further determine the display type corresponding to the guide contents based on the preset rule (e.g., adjusting a color of the text information corresponding to the function that need to be performed in each interface to red). As another example, in addition to the operating contents, the processor 110 may analyze and process the guide information to determine the count of processes that need to be performed in the target operating interface, the preset rule includes that when the current count of processes is less than a preset threshold, the processor 110 may determine that the display type of the operating guide information is text guidance, the operating guide information includes displaying specific operations in the corresponding target operating interface through the text, the operation processes and operations may be determined based on the content corresponding to the target operating interface in the guide information; when the current count of processes is not less than the preset threshold, the processor 110 may determine that the display type of the operating guide information is the image guidance or animation guidance. The operating guide information may display specific operation processes in the corresponding target operating interface through the images or animation.

In some embodiments, the processor 110 may also determine the display type of operating guide information in other ways. For example, the processor 110 may determine a spatial size of a blank region in the target operating interface through image recognition techniques (e.g., segmenting the blank region and a content region of the target operating interface based on a convolutional neural network model), and determine the display type and guide content of the operating guide information in the target operating interface based on the guide information and the spatial size. A relatively small space is suitable for display the sample guide information, the display type is the text guidance; a relatively big space is suitable for display the complex guide information, the display type is the image or animation guidance. As another example, the processor 110 may also cross guide through the multiple display types based on the complexity of the guide information, wherein the processor 110 may analyze and process the guide information to determine the count of processes that need to be performed in the target operating interface, and based on the count of processes, the complexity of the guide information may be determined by a preset corresponding relationship.

In some embodiments, after the user completes the corresponding parameter settings related to the target task based on the operating guide information, the apparatus 150 may send the corresponding parameters to the medical device 160 and send a startup instruction to start the medical device 160 to complete the corresponding target task. For example, after completing the parameter settings related to the target task in the target operating interface, the user may click a corresponding trigger button (e.g., start) to send the start instruction of the target task to the medical device 160.

For example, when the operating guide information includes a start control corresponding to the target task, the apparatus 150 may generate the start instruction of the target task based on trigger information corresponding to the triggering operation of the start control of the target task of the user, send the start instruction to the medical device 160 through wired or wireless communication, and control the medical device 160 to perform the start instruction of the target task to complete the target task. As another example, when the operating guide information includes a start control corresponding to the target task, the apparatus 150 may jump to the target operating interface including the triggering operation in response to receiving the triggering operation of the start control of the target task of the user, and control the medical device 160 to perform the start instruction of the target task in the target operating interface to complete the target task based on the triggering operation.

In some embodiments, the second information feedback interface of the target operating interface may also be used to display a scanning result of the medical device, an image acquisition result (e.g., a medical images), or the like. In some embodiments, the processor may obtain a window thumbnail of a window to be displayed, the window thumbnail is configured to represent a custom layout of each sequence window in the window to be displayed; display the window to be displayed and the sequence window in the window to be displayed in the second information feedback interface according to the window thumbnail; and the sequence window is configured to browse a medical image of a corresponding sequence.

In some embodiments of the present disclosure, based on a size, an interface setting, and a difficulty level of guide information for target operating interfaces of different medical devices, the operating guide information may be displayed by various display types, which can be more compatible with the target operating interface of the medical device, and displaying the operating guide information can enable the user to perform corresponding operations more clearly and clearly, improve the operational efficiency.

In some embodiments, the target operating interface may be displayed by the user triggering, the displayed type of the operating guide information may be determined based on a specific target operating interface and a specific content of the guide information, and the user may be guided in completing the target task. By this manner, a suitable display form may be determined for the target operating interfaces of different sizes and settings. The guide information with different difficulty levels may be displayed through different display types, which can provide the user with more intuitive and flexible guidance, improve the efficiency of medical device use, and reduce operational errors.

The present disclosure provides an apparatus for guiding an operation of a medical device, comprising at least one processor and at least one storage, wherein the at least one storage is configured to store computer instructions, and the at least one processor is configured to execute at least part of the computer instructions to implement the method for guiding the operation of the medical device of any one of claims.

The present disclosure provides computer readable storage medium, storing computer instructions, wherein when executed by a computer, the computer instructions cause the computer to implement the method for guiding the operation of the medical device of any one of claims.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of the specification are not necessarily all referring to the same embodiment. In addition, some features, structures, or features in the present disclosure of one or more embodiments may be appropriately combined.

Moreover, unless otherwise specified in the claims, the sequence of the processing elements and sequences of the present application, the use of digital letters, or other names are not used to define the order of the application flow and methods. For example, although the implementation of various assemblies described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure and aiding in the understanding of one or more of the various embodiments. However, this disclosure may not mean that the present disclosure subject requires more features than the features mentioned in the claims. In fact, the features of the embodiments are less than all of the features of the individual embodiments disclosed above.

In some embodiments, the numbers expressing quantities, properties, and so forth, used to describe and claim certain embodiments of the application are to be understood as being modified in some instances by the term "about," "approximate," or "substantially." Unless otherwise stated, "about," "approximate," or "substantially" may indicate a ±20% variation of the value it describes. Accordingly, in some embodiments, the numerical parameters set forth in the description and attached claims are approximations that may vary depending upon the desired properties sought to be obtained by a particular embodiment. In some embodiments, the numerical parameters should be construed in light of the number of reported significant digits and by applying ordinary rounding techniques. Although the numerical domains and parameters used in the present application are used to confirm the range of ranges, the settings of this type are as accurate in the feasible range in the feasible range in the specific embodiments.

**In** addition to the application history documents that are inconsistent or conflicting with the contents of the present disclosure, the documents that may limit the widest range of the claim of the present disclosure (currently or later attached to this application) are excluded from the present disclosure. It should be noted that if the description, definition, and/or terms used in the appended application of the present disclosure is inconsistent or conflicting with the content described in the present disclosure, the use of the description, definition and/or terms of the present disclosure shall prevail.

## Claims

1. A method for guiding an operation of a medical device, wherein the medical device refers to a related device used to perform medical tasks, comprising:
obtaining (310) retrieving information input by a user, wherein the retrieving information includes a corresponding target task, the target task refers to a relevant task that the user needs to understand and includes at least one of a general knowledge retrieving task related to the medical device, an image acquisition task, and a fault detection task related to the medical device; and
determining (320) at least one piece of guide information corresponding to the retrieving information based on the retrieving information, wherein the at least one piece of guide information is configured to guide the user in completing the target task, the at least one piece of guide information includes at least one of an operation instruction, an operation process, an operation guideline, an operation prompt, or an operation demonstration; **characterized in that** when the at least one piece of guide information includes multiple pieces of guide information, the method further includes:
obtaining at least one of historical operation record of the user, user information, and a current scanning body part; and
determining a sequence number for each piece of guide information in the multiple pieces of guide information based on the multiple pieces of guide information and the at least one of the historical operation record, the user information, and the current scanning body part.

2. The method of claim 1, wherein the retrieving information includes at least one of text information, voice information, and image information related to the target task.

3. The method of claim 2, wherein when the retrieving information includes the text information and/or the voice information, determining the at least one piece of guide information corresponding to the retrieving information based on the retrieving information includes:
determining (610) a retrieving keyword of the retrieving information by processing the retrieving information; and
determining (620) the at least one piece of guide information corresponding to the retrieving information based on the retrieving keyword.

4. The method of any one of claims 1-3, wherein the determining the at least one piece of guide information corresponding to the retrieving information based on the retrieving information includes:
determining the at least one piece of guide information corresponding to the retrieving information by processing the retrieving information based on a retrieval analysis model that is a machine learning model.

5. The method of any one of claims 1-4, wherein the target task includes at least one of a general knowledge retrieving task, an image acquisition task, and a fault detection task.

6. The method of claim 5, wherein the determining the at least one piece of guide information corresponding to the retrieving information based on the retrieving information includes:
determining (810) a task type of the target task based on the retrieving information;
in response to determining that the task type of the target task is the fault detection task, obtaining (820) a fault interface and one or more system logs of the medical device used by the user; and
determining (830) the at least one piece of guide information corresponding to the retrieving information based on the retrieving information, the fault interface, and the system log.

7. The method of any one of claims 1-6, wherein the method further includes:
in response to a triggering operation of the user, displaying a target operating interface, wherein the target operating interface is related to the at least one piece of guide information.

8. The method of claim 7, wherein the target operating interface includes operating guide information, the operating guide information is configured to guide the user in completing a corresponding operation on the target operating interface, the method further includes:
guiding the user in operating on the target operating interface to complete the target task based on the operating guide information.

9. The method of claim 8, wherein a displaying type of the operating guide information includes at least one of a text guidance, an image guidance, and an animation guidance.

10. A system for guiding an operation of a medical device, wherein the medical device refers to a related device used to perform medical tasks, comprising:
an obtaining module (210), configured to obtain (310) retrieving information input by a user, wherein the retrieving information includes a corresponding target task, the target task refers to a relevant task that the user needs to understand and includes at least one of a general knowledge retrieving task related to the medical device, an image acquisition task, and a fault detection task related to the medical device; and
a processing module (220), configured to determine (320) at least one piece of guide information corresponding to the retrieving information based on the retrieving information, wherein the at least one piece of guide information is configured to guide the user in completing the target task, the at least one piece of guide information includes at least one of an operation instruction, an operation process, an operation guideline, an operation prompt, or an operation demonstration; **characterized in that** when the at least one piece of guide information includes multiple pieces of guide information, the processing module (220) further
obtain at least one of historical operation record of the user, user information, and a current scanning body part; and
determine a sequence number for each piece of guide information in the multiple pieces of guide information based on the multiple pieces of guide information and the at least one of the historical operation record, the user information, and the current scanning body part.

11. The system of claim 10, wherein the retrieving information includes at least one of text information, voice information, and image information related to the target task.

12. The system of claim 11, wherein when the retrieving information includes the text information and/or the voice information, the processing module is further configured to:
determine (610) a retrieving keyword of the retrieving information by processing the retrieving information; and
determine (620) the at least one piece of guide information corresponding to the retrieving information based on the retrieving keyword.

13. An apparatus for guiding an operation of a medical device, wherein the device includes at least one processor (110) and at least one memory (130), the at least one memory (130) is configured to store computer instructions, the at least one processor (110) is configured to execute at least part instructions of the computer instructions to implement the method for guiding the operation of the medical device of any one of claims 1-9.

14. A computer readable storage medium, the storage medium stores computer instructions, when the computer executes the computer instructions, the method for guiding the operation of the medical device of any one of claims 1-9 is implemented.

## Patentansprüche

1. Verfahren zum Anleiten einer Bedienung einer medizinischen Vorrichtung, wobei sich die medizinische Vorrichtung auf eine zugehörige Vorrichtung bezieht, die verwendet wird, um medizinische Aufgaben durchzuführen, umfassend:
Erhalten (310) von Abrufinformationen, die von einem Benutzer eingegeben werden, wobei die Abrufinformationen eine entsprechende Zielaufgabe beinhalten, wobei sich die Zielaufgabe auf eine relevante Aufgabe bezieht, die der Benutzer verstehen muss, und mindestens eines von einer allgemeinen Wissensabrufaufgabe in Bezug auf die medizinische Vorrichtung, eine Bildaufnahmeaufgabe und eine Fehlererkennungsaufgabe in Bezug auf die medizinische Vorrichtung beinhaltet; und
Bestimmen (320) mindestens einer Anleitungsinformation, die den Abrufinformationen entspricht, basierend auf den Abrufinformationen, wobei die mindestens eine Anleitungsinformation so konfiguriert ist, dass sie den Benutzer anleitet, die Zielaufgabe abzuschließen, die mindestens eine Anleitungsinformation mindestens eines von einer Bedienungsanweisung, einem Bedienungsprozess, einer Bedienungsrichtlinie, einer Bedienungsaufforderung oder einer Bedienungsdemonstration beinhaltet; **dadurch gekennzeichnet, dass**
wenn die mindestens eine Anleitungsinformation mehrere Anleitungsinformationen beinhaltet, das Verfahren ferner beinhaltet:
Erhalten mindestens eines von einer historischen Bedienungsaufzeichnung des Benutzers, Benutzerinformationen und einem aktuell abgetasteten Körperteil; und
Bestimmen einer Sequenznummer für jede Anleitungsinformation unter den mehreren Anleitungsinformationen basierend auf den mehreren Anleitungsinformationen und dem mindestens einen von der historischen Bedienungsaufzeichnung, den Benutzerinformationen und dem aktuell abgetasteten Körperteil.

2. Verfahren nach Anspruch 1, wobei die Abrufinformationen mindestens eine von Textinformationen, Sprachinformationen und Bildinformationen bezüglich der Zielaufgabe beinhalten.

3. Verfahren nach Anspruch 2, wobei, wenn die Abrufinformationen die Textinformationen und/oder die Sprachinformationen beinhalten, Bestimmen der mindestens einen Anleitungsinformation entsprechend der Abrufinformationen, basierend auf den Abrufinformationen, beinhaltet:
Bestimmen (610) eines Abrufschlüsselworts der Abrufinformationen durch Verarbeiten der Abrufinformationen; und
Bestimmen (620) der mindestens einen Anleitungsinformation entsprechend den Abrufinformationen, basierend auf dem Abfrufschlüsselwort.

4. Verfahren nach einem der Ansprüche 1-3, wobei das Bestimmen der mindestens einen Anleitungsinformation entsprechend den Abrufinformationen, basierend auf den Abrufinformationen, beinhaltet:
Bestimmen der mindestens einen Anleitungsinformation entsprechend den Abrufinformationen durch Verarbeiten der Abrufinformationen basierend auf einem Abrufanalysemodell, das ein maschinelles Lernmodell ist.

5. Verfahren nach einem der Ansprüche 1-4, wobei die Zielaufgabe mindestens eine allgemeine Wissensabrufaufgabe, eine Bildaufnahmeaufgabe und eine Fehlererkennungsaufgabe beinhaltet.

6. Verfahren nach Anspruch 5, wobei das Bestimmen der mindestens einen Anleitungsinformation entsprechend den Abrufinformationen, basierend auf den Abrufinformationen, beinhaltet:
Bestimmen (810) eines Aufgabentyps der Zielaufgabe basierend auf den Abrufinformationen;
infolge eines Bestimmens, dass der Aufgabentyp der Zielaufgabe der Zielaufgabe die Fehlererkennungsaufgabe ist, Erhalten (820) einer Fehlerschnittstelle und eines oder mehrerer Systemprotokolle der vom Benutzer verwendeten medizinischen Vorrichtung; und
Bestimmen (830) der mindestens einen Anleitungsinformation entsprechend den Abrufinformationen, basierend auf dem Abfrufschlüsselwort, der Fehlerschnittstelle und dem Systemprotokoll.

7. Verfahren nach einem der Ansprüche 1-6, wobei das Verfahren ferner beinhaltet:
infolge einer Auslösebedienung des Benutzers, Anzeigen einer Zielbedingungsschnittstelle, wobei die Zielbedienungsschnittstelle sich auf die mindestens eine Anleitungsinformation bezieht.

8. Verfahren nach Anspruch 7, wobei die Zielbedienungsschnittstelle Bedienungsanweisungsinformationen beinhaltet, wobei die Bedienungsanweisungsinformationen so konfiguriert sind, dass sie den Benutzer anleiten, eine entsprechende Bedienung auf der Zielbedienungsschnittstelle abzuschließen, wobei das Verfahren ferner beinhaltet:
Anleiten des Benutzers beim Bedienen der Zielbedienungsschnittstelle, um die Zielaufgabe basierend auf den Bedienungsanleitungsinformationen abzuschließen.

9. Verfahren nach Anspruch 8, wobei eine Anzeigeart der Bedienungsanleitungsinformationen mindestens eine von einer Textanleitung, einer Bildanleitung und einer Animationsanleitung beinhaltet.

10. System zum Anleiten einer Bedienung einer medizinischen Vorrichtung, wobei sich die medizinische Vorrichtung auf eine zugehörige Vorrichtung bezieht, die verwendet wird, um medizinische Aufgaben durchzuführen, umfassend:
ein Erhaltungsmodul (210) das zum Erhalten (310) von Abrufinformationen konfiguriert ist, die von einem Benutzer eingegeben werden, wobei die Abrufinformationen eine entsprechende Zielaufgabe beinhalten, die Zielaufgabe sich auf eine relevante Aufgabe bezieht, die der Benutzer verstehen muss, und mindestens eines von einer allgemeinen Wissensabrufaufgabe in Bezug auf die medizinische Vorrichtung, eine Bildaufnahmeaufgabe und eine Fehlererkennungsaufgabe in Bezug auf die medizinische Vorrichtung beinhaltet; und
ein Verarbeitungsmodul (220), das zum Bestimmen (320) mindestens einer Anleitungsinformation konfiguriert ist, die den Abrufinformationen entspricht, basierend auf den Abrufinformationen, wobei die mindestens eine Anleitungsinformation so konfiguriert ist, dass sie den Benutzer anleitet, die Zielaufgabe abzuschließen, die mindestens eine Anleitungsinformation mindestens eines von einer Bedienungsanweisung, einem Bedienungsprozess, einer Bedienungsrichtlinie, einer Bedienungsaufforderung oder einer Bedienungsdemonstration beinhaltet; **dadurch gekennzeichnet, dass**
wenn die mindestens eine Anleitungsinformation mehrere Anleitungsinformationen beinhaltet, das Verarbeitungsmodul (220) ferner
mindestens eines von einer historischen Bedienungsaufzeichnung des Benutzers, Benutzerinformationen und einem aktuell abgetasteten Körperteil erhält; und
eine Sequenznummer für jede Anleitungsinformation unter den mehreren Anleitungsinformationen basierend auf den mehreren Anleitungsinformationen und dem mindestens einen von der historischen Bedienungsaufzeichnung, den Benutzerinformationen und dem aktuell abgetasteten Körperteil bestimmt.

11. System nach Anspruch 10, wobei die Abrufinformationen mindestens eine von Textinformationen, Sprachinformationen und Bildinformationen bezüglich der Zielaufgabe beinhalten.

12. System nach Anspruch 11, wobei, wenn die Abrufinformationen die Textinformationen und/oder die Sprachinformationen beinhalten, das Verarbeitungsmodul ferner konfiguriert ist zum:
Bestimmen (610) eines Abrufschlüsselworts der Abrufinformationen durch Verarbeiten der Abrufinformationen; und
Bestimmen (620) der mindestens einen Anleitungsinformation entsprechend den Abrufinformationen, basierend auf dem Abfrufschlüsselwort.

13. Einrichtung zum Anleiten einer Bedienung einer medizinischen Vorrichtung, wobei die Vorrichtung mindestens einen Prozessor (110) und mindestens einen Speicher (130) beinhaltet, wobei der mindestens eine Speicher (130) dazu konfiguriert ist, Computeranweisungen zu speichern, wobei der mindestens eine Prozessor (110) dazu konfiguriert ist, mindestens Teilanweisungen der Computeranweisungen auszuführen, um das Verfahren zum Anleiten der Bedienung der medizinischen Vorrichtung nach einem der Ansprüche 1-9 zu implementieren.

14. Computerlesbares Speichermedium, wobei das Speichermedium Computeranweisungen speichert, wobei, wenn der Computer die Computeranweisungen ausführt, das Verfahren zum Anleiten der Bedienung der medizinischen Vorrichtung nach einem der Ansprüche 1-9 implementiert wird.

## Revendications

1. Procédé de guidage d'une utilisation d'un dispositif médical, dans lequel le dispositif médical désigne un dispositif connexe utilisé pour effectuer des tâches médicales, comprenant :
l'obtention (310) d'informations de récupération saisies par un utilisateur, dans lequel les informations de récupération incluent une tâche cible correspondante, la tâche cible fait référence à une tâche pertinente que l'utilisateur doit comprendre et inclut au moins une tâche parmi une tâche de récupération de connaissances générales relative au dispositif médical, une tâche d'acquisition d'images et une tâche de détection de défauts relative au dispositif médical ; et
la détermination (320) d'au moins un élément d'information de guidage correspondant aux informations de récupération sur la base des informations de récupération, dans lequel l'au moins un élément d'information de guidage est configuré pour guider l'utilisateur dans l'accomplissement de la tâche cible, l'au moins un élément d'information de guidage inclut au moins un élément parmi une instruction d'utilisation, un processus d'utilisation, une directive d'utilisation, une invite d'utilisation ou une démonstration d'utilisation ; **caractérisé en ce que**
lorsque l'au moins un élément d'information de guidage inclut de multiples éléments d'information de guidage, le procédé inclut en outre :
l'obtention d'au moins un élément parmi un historique d'utilisation de l'utilisateur, des informations d'utilisateur et une partie du corps actuellement scannée ; et
la détermination d'un numéro de séquence pour chaque élément d'information de guidage dans les multiples éléments d'information de guidage sur la base des multiples éléments d'information de guidage et de l'au moins un des éléments parmi l'historique d'utilisation, les informations d'utilisateur et la partie du corps actuellement scannée.

2. Procédé selon la revendication 1, dans lequel les informations de récupération incluent au moins une information parmi des information textuelles, des informations vocales et des informations d'image liées à la tâche cible.

3. Procédé selon la revendication 2, dans lequel lorsque les informations de récupération incluent les informations textuelles et/ou les informations vocales, la détermination de l'au moins un élément d'information de guidage correspondant aux informations de récupération sur la base des informations de récupération inclut :
la détermination (610) d'un mot-clé de récupération des informations de récupération en traitant les informations de récupération ; et
la détermination (620) d'au moins un élément d'information de guidage correspondant aux informations de récupération sur la base du mot-clé de récupération.

4. Procédé selon l'une quelconque des revendications 1-3, dans lequel la détermination de l'au moins un élément d'information de guidage correspondant aux informations de récupération sur la base des informations de récupération inclut :
la détermination d'au moins un élément d'information de guidage correspondant aux informations de récupération en traitant les informations de récupération sur la base d'un modèle d'analyse de récupération qui est un modèle d'apprentissage automatique.

5. Procédé selon l'une quelconque des revendications 1-4, dans lequel la tâche cible inclut au moins une tâche parmi une tâche de récupération de connaissances générales, une tâche d'acquisition d'images et une tâche de détection de défauts.

6. Procédé selon la revendication 5, dans lequel la détermination de l'au moins un élément d'information de guidage correspondant aux informations de récupération sur la base des informations de récupération inclut :
la détermination (810) d'un type de tâche de la tâche cible sur la base des informations de récupération ;
en réponse à la détermination que le type de tâche de la tâche cible est la tâche de détection de défauts, l'obtention (820) d'une interface de défaut et d'un ou plusieurs journaux système du dispositif médical utilisé par l'utilisateur ; et
la détermination (830) de l'au moins un élément d'information de guidage correspondant aux informations de récupération sur la base des informations de récupération, de l'interface de défaut et du journal système.

7. Procédé selon l'une quelconque des revendications 1-6, dans lequel le procédé inclut en outre :
en réponse à une utilisation de déclenchement de l'utilisateur, l'affichage d'une interface d'utilisation cible, dans lequel l'interface d'utilisation cible est liée à l'au moins un élément d'information de guidage.

8. Procédé selon la revendication 7, dans lequel l'interface d'utilisation cible inclut des informations de guidage d'utilisation, les informations de guidage d'utilisation sont configurées pour guider l'utilisateur dans l'accomplissement d'une utilisation correspondante sur l'interface d'utilisation cible, le procédé inclut en outre :
le guidage de l'utilisateur dans l'utilisation de l'interface d'utilisation cible pour accomplir la tâche cible sur la base des informations de guidage d'utilisation.

9. Procédé selon la revendication 8, dans lequel un type d'affichage des informations de guidage d'utilisation inclut au moins un guide parmi un guide textuel, un guide d'image et un guide d'animation.

10. Système de guidage d'une utilisation d'un dispositif médical, dans lequel le dispositif médical fait référence à un dispositif connexe utilisé pour effectuer des tâches médicales, comprenant :
un module d'obtention (210), configuré pour obtenir (310) des informations de récupération saisies par un utilisateur, dans lequel les informations de récupération incluent une tâche cible correspondante, la tâche cible fait référence à une tâche pertinente que l'utilisateur doit comprendre et inclut au moins une tâche parmi une tâche de récupération de connaissances générales relative au dispositif médical, une tâche d'acquisition d'images et une tâche de détection de défaut relative au dispositif médical ; et
un module de traitement (220), configuré pour déterminer (320) au moins un élément d'information de guidage correspondant aux informations de récupération sur la base des informations de récupération, dans lequel l'au moins un élément d'information de guidage est configuré pour guider l'utilisateur dans l'accomplissement de la tâche cible, l'au moins un élément d'information de guidage inclut au moins une parmi un élément parmi une instruction d'utilisation, un processus d'utilisation, une directive d'utilisation, une invite d'utilisation ou une démonstration d'utilisation ; **caractérisé en ce que**
lorsque l'au moins un élément d'information de guidage inclut de multiples informations de guidage, le module de traitement (220)
obtient en outre au moins un élément parmi un historique d'utilisation de l'utilisateur, des informations d'utilisateur et une partie du corps actuellement scannée ; et
détermine un numéro de séquence pour chaque élément d'information de guidage dans les multiples éléments d'information de guidage sur la base des multiples éléments d'information de guidage et de l'au moins un élément parmi l'historique d'utilisation, les informations d'utilisateur et la partie du corps actuellement scannée.

11. Système selon la revendication 10, dans lequel les informations de récupération incluent au moins une information parmi des informations textuelles, des informations vocales et des informations d'image liées à la tâche cible.

12. Système selon la revendication 11, dans lequel lorsque les informations de récupération incluent des informations textuelles et/ou des informations vocales, le module de traitement est en outre configuré pour :
déterminer (610) un mot-clé de récupération des informations de récupération en traitant les informations de récupération ; et
déterminer (620) l'au moins un élément d'information de guidage correspondant aux informations de récupération sur la base du mot-clé de récupération.

13. Appareil de guidage d'une utilisation d'un dispositif médical, dans lequel le dispositif inclut au moins un processeur (110) et au moins une mémoire (130), l'au moins une mémoire (130) est configurée pour stocker des instructions informatiques, l'au moins un processeur (110) est configuré pour exécuter au moins une partie des instructions des instructions informatiques pour mettre en œuvre le procédé de guidage de l'utilisation du dispositif médical selon l'une quelconque des revendications 1-9.

14. Support de stockage lisible par ordinateur, le support de stockage stocke des instructions d'ordinateur, lorsque l'ordinateur exécute les instructions d'ordinateur, le procédé de guidage de l'utilisation du dispositif médical selon l'une quelconque des revendications 1-9 est mis en œuvre.
